(19)

Europäisches
Patentamt
European
Patent Office
Office européen
des brevets

(11) **EP 2 365 458 B1**

(12) **EUROPEAN PATENT SPECIFICATION**

(45) Date of publication and mention
of the grant of the patent:
**01.11.2017 Bulletin 2017/44**

(51) Int Cl.:
***G06F 19/00*** (2011.01)  ***G06F 21/62*** (2013.01)
***H04L 9/08*** (2006.01)

(21) Application number: **11157135.2**

(22) Date of filing: **07.03.2011**

(54) **A computer implemented method for determining the presence of a disease in a patient**

Computerimplementiertes Verfahren zur Erzeugung eines Pseudonyms, computerlesbares
Speichermedium und Computersystem

Procédé implémenté informatique pour générer un pseudonyme, support de stockage lisible sur
ordinateur et système informatique

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.03.2010 EP 10156171
29.06.2010 EP 10167641
18.08.2010 EP 10173163
18.08.2010 EP 10173175
18.08.2010 EP 10173198
13.12.2010 EP 10194677
13.12.2010 EP 10194681
13.12.2010 EP 10194686**

(43) Date of publication of application:
**14.09.2011 Bulletin 2011/37**

(73) Proprietors:
• **CompuGroup Medical SE
56070 Koblenz (DE)**
• **Spalka, Adrian
56076 Koblenz (DE)**

(72) Inventor: **Gotthardt, Frank
56337, Eitelborn (DE)**

(74) Representative: **Richardt Patentanwälte PartG
mbB
Wilhelmstraße 7
65185 Wiesbaden (DE)**

(56) References cited:
**WO-A1-02/09580    WO-A2-00/41613
WO-A2-01/69513    US-A1- 2006 153 370**

• **M. A. Musen et al: "Clinical Decision-Support
Systems" In: SHORTLIFFE E H ET AL:
"Biomedical informatics : computer applications
in health care and biomedicine", 25 May 2006
(2006-05-25), BIOMEDICAL INFORMATICS :
COMPUTER APPLICATIONS IN HEALTH CARE
AND BIOMEDICINE; [HEALTH INFORMATICS
SERIES], NEW YORK, NY : SPRINGER, US,
PAGE(S) 698-736, XP002570368, ISBN:
978-0-387-28986-1 * page 698 - page 736 ***

Remarks:
The file contains technical information submitted after
the application was filed and not included in this
specification

**Description**

**Field of the invention**

The present invention relates to the field of computer implemented methods for determining the presence of a disease in a patient.

**Background and related art**

Various computer implemented schemes exist for determining whether a disease is present in a patient or not. Such schemes are commonly implemented in the form of decision support systems (DSSs) which assist a physician in making diagnoses and treatment related decisions. Said diagnoses and decisions typically depend on a rapidly growing amount of biomedical knowledge.

A common problem of current DSS is, that the data basis on which a decision is made, i.e. patient-related medical data, is often distributed among multiple independently practicing physicians and hospitals. As a consequence, often only a fraction of the theoretically available biomedical data is used by current DSS which are typically implemented as stand-alone software applications running on the computer systems in doctors' practices or in hospitals. As a consequence, the data basis actually used for determining a diagnosis by means of current DSSs tends to be small. This has a negative impact on the prediction quality.

Said problem is particularly severe for diseases which affect multiple organ systems at the same time because a patient may visit a physician who is specialized in only one particular organ system. Said physician may not be able to recognize a disease which primarily affects other organ systems. This drawback has also a negative impact on the prediction quality.

A disease may be particularly hard to detect/diagnose if said disease affects different organ system to a different degree in different patients. The disease Morbus Fabry, for example, is known for potentially affecting multiple organ systems such as the nervous system (pain episodes), the renal system (kidney complications), the cardiac system (hypertension and cardiomyopathy) and other organ system. Some Morbus Fabry patients may be affected by pain, cardial and renal problems at the same time while others may merely be affected by cardiac problems ('cardiac type' of Morbus Fabry) or by renal problems ('renal type' of Morbus Fabry). Diseases affecting multiple organ systems to a different degree in different patients are particularly difficult to detect by a human or a DSS when merely/primarily symptoms of one single organ system are considered. This, however, is often the case, because many physicians have specialized in a particular medical sub-discipline and because many DSSs in practice operate on biomedical data which does not sufficiently cover biomedical data of all organ systems of a patient.

Bringing medical data having been created by a multitude of different, independently practicing physicians and hospitals together is often, however, not possible due to a multitude of problems regarding the compatibility of data formats and due to problems regarding the security of a centralized data repository comprising sensitive, biomedical data of one or more patients.

According to the free encyclopedia Wikipedia, an 'orphan disease' is a disease that affects only a small percentage of the population. There is no single, widely accepted definition for rare diseases. In the United States, the Rare Disease Act of 2002 defines rare disease strictly according to prevalence, specifically as "any disease or condition that affects less than 200,000 persons in the United States", which is about 1 in 1,500 people. In Japan, the legal definition of a rare disease is one that affects fewer than 50,000 patients in Japan, or about 1 in 2,500 people. The automated prediction of orphan diseases is particularly difficult for the following reasons: every prediction method produces false positive and false negative errors. The higher the accuracy of a prediction method, the smaller the fraction of false positive and/or false-negative results. In case a decision support system generates one false-positive results when evaluating the medical records of 1000 patients, said number is usually considered as highly accurate for predicting frequently occurring diseases. When an orphan disease of an occurrence of one in 200,000 persons has to be diagnosed automatically by said method, however, 200 persons would erroneously be predicted to be affected by said rare disease, while statistically only one true positive result should be expected. Using a highly accurate prediction method is therefore particularly important when an orphan disease is to be automatically determined by a decision support system.

U.S. 2007/0112782 A1 discloses a DSS system providing access to medical knowledge in a machine-executable format. The system is network-based and is based on executable knowledge modules (EKMs). Said modules specify the data to be used for calculating a decision and the conclusions that can be drawn in the light of these data. The system includes a network server that communicates with a client; the client may include one or more client applications or data sources.

U.S. 2008/0263050 A1 relates to a content management system being operable to manage patient related data such as prescriptions, medication dose, dates of medical monitoring equipment, or drug dosages. The system includes a database for storing the patient data, a database server and at least one client system, which is connected to the server. The system is operable to receive data from various sources such as a fax, a scanner or from authorized persons, such as doctors, nurses, pharmacist via a man-machine interface. The system is in addition operable to submit an alarm message, preferably via an encrypted e-mail, to the client in dependence on the data.

WO 2008/085881 A1 relates to a machine for the integration of medical guidelines. These guidelines are imported into a knowledge base from different data sources which may each be based on a different data format.

Various other DSS variants are described e.g. in U.S. 2002/091687 A1, U.S. 2009/0187419 A1, U.S. 2010/0131296 A1, U.S. 2010/0088320 A1, US7742932, US006754655 and US006212519. European patent application 09179974 which is hereby included by reference discloses a computer-implemented method for generating a pseudonym.

WO 00/41613 A2 describes an expert system for real-time decision support for performing individual point-of-care diagnostics and treatment using expert decision-making processes. The system is particularly well-suited to the medical industry. The expert system comprises an assessment system for receiving input data about a patient's health status; a problem identification system for analyzing the input data against the problem identification healthcare knowledge base and associating a probable diagnosis based on the analysis of the input data; a desired outcome generation system for analyzing the probable diagnosis against a desired outcome healthcare knowledge base and associating a desired outcome based on the analysis of the probable diagnosis.

The publication entitled "Biomedical Data: the Acquisition, Storage, and Use" by Edward H. Shortliffe and G. Octo Barnett, XP-002570368, 25 May 2006 in Biomedical Informatics: Computer Applications in Healthcare and Biomedicine; [Health Informatics Series], New York, Springer, pages 698-736, ISBN: 978-0-387-28986-1 various aspects of clinical decision support systems, e.g. requirements for an excellent decision-making system, what influences account for the gradual improvement of clinical decision support systems and other aspects.

US 2006/153370 A1 describes a method of providing a public-private key pair by: receiving into a computer system input data from a user (UID); generating within the computer system the first key as a deterministic function of the UID; and generating within the computer system a second key as a deterministic function of the first key. The first key is the private key and the second key is the public key. The first key is cleared from the computer system following generation of the second key. Neither the UID nor the first key is exported from the computer system. The second key may be exported from the computer system.

**Summary**

The invention provides for a computer readable storage medium, a computer implemented method, and a computer system in the independent claims. Embodiments are given in the dependent claims.

Definitions

The term 'disease indicator' will in the following encompass any parameter correlating with, causing or being the effect of a particular disease. A disease indicator can be, for example, a symptom, a finding, a complaint, billing data, a medication or treatment. It may also encompass parameters characterizing the medical or biological history of a patient such as surgeries, pregnancies, smoking habits, nutritional habits, the age or the gender of a patient. A disease indicator may be a laboratory value having been measured on a sample of the patient, e.g. the blood glucose level of a patient. A disease indicator may also be a parameter being indicative of diseases having affected relatives. A disease indicator may also be a comorbid condition and data being indicative of the presence of a secondary or tertiary illness. Some or all disease indicators may be represented as free text or by means of a standard, e.g. a biomedical ontology or catalog such as the ICD 10 code for Germany. Depending on the respective country or application scenario, different standards and codes may be used for encoding disease indicators.

'Biomedically' related disease indicators are disease indicators which relate to a common physiological, genetic and/or metabolic cause or which have at least been observed to positively or inversely correlate with each other. 'Biomedically' related disease indicators comprise disease indicators affecting e.g. the same organ system. As medical sub-disciplines such as cardiology, neurology or orthopedics typically relate to one or more particular organ systems, disease indicators considered as relevant in a particular medical sub-discipline are in the following therefore also considered as disease indicators being 'biomedically related' to each other. For example, it is known that disease indicators such diverse as near-work (e.g. reading) in childhood and teenage years, genetic factors as well as environmental factors (e.g. pesticides) may contribute to the development of myopia. As all said parameters appear to affect a particular biologic function and organ system (the eye) and are known to have an impact on the development of a particular disease (myopia), said parameters are herein also considered as 'biomedically related' disease indicators. Preferentially, however, 'biomedically' related disease indicators are disease indicators of the same organ system.

A 'rule' is a computer-interpretable statement with two parts: an if-clause and a then-clause, whereby the then-clause is only executed in case the if-clause returns the Boolean value 'true'. Depending on the embodiment, a rule may be implemented e.g. as a set of one or more conditions in the if-clause part having been stored in a rules repository.

Any kind of data structure storing rules and being accessible by a rule engine will in the following be referred to as 'rule repository'. Depending on the embodiment, a rule repository may be implemented as a (e.g. relational) database, as a directory comprising one or more files or comprising serialized data objects whereby said files or serialized data objects comprise the rules. A rule engine is any kind of computer implemented logic, in particular a software module, being operable to read one or more rules from the rules repository and execute the read rules on biomedical patient data.

'Biomedical data' of a patient as used herein encompasses any kind of clinically or medically relevant data having been measured, queried or otherwise retrieved from a patient. In particular, biomedical data of a patient comprises data values of disease indicators (medical history, ICD 10 codes, age, gender, etc.).

A 'doctor information system', also known as 'medical information system', is any kind of program logic, in particular software-based program logic, providing a computer-based information storage, retrieval, and analysis system for personal and biomedical data of a patient, e.g. addresses, medication history, treatments, diagnoses, and the like. Typically, a 'doctor information system' is installed on a computer system used by a medical practitioner.

The term 'user-selected secret' is understood herein as any secret data that is selected by or related to a user, such as a user-selected secret password or a secret key, such as a symmetric cryptographic key. Further, the term 'user-selected secret' does also encompass a combination of biometric data obtained from the user and a user-selected password or secret key, such as a biometric hash value of the password or secret key.

The term 'memory' as used herein encompasses any volatile or non-volatile electronic memory component or a plurality of electronic memory components, such as a random access memory.

The term 'embedding function' or 'embedding component' as used herein encompasses any injective function that maps the elements of an n-dimensional space onto elements of an m-dimensional space, where $n > m$. For the purpose of this invention, we focus on embedding functions where $m = 1$. In accordance with embodiments of this invention n is equal to 2 and m is equal to 1 for combining two elements onto a single element. In one embodiment, a user-selected secret and a public parameter are mapped by the embedding function to the 1-dimensional space to provide a combination of the user selected secret and the public parameter, e.g. a single number that embeds the user selected secret and the public parameter. This single number constitutes the embedded secret. In another embodiment, a first hash value of the user selected secret and a random number are mapped by the embedding function to the 1-dimensional space to provide the embedded secret.

A 'randomizing function' or 'randomizing component' as understood herein encompasses any injective function that provides an output of data values that are located within a predefined interval and wherein the distribution of the data values within the predefined interval is a substantially uniform distribution.

The term 'embedding and randomizing function' as used herein encompasses any function that implements both an embedding function and a randomizing function.

The term 'computer readable storage medium' as used herein encompasses any storage medium which may store instructions which are executable by a processor of a computing device. In some embodiments, a computer readable storage medium may also be able to store data which is able to be accessed by the processor of the computing device. An example of a computer readable storage medium include, but are not limited to: a floppy disk, a magnetic hard disk drive, a solid state hard disk, flash memory, a USB thumb drive, Random Access Memory (RAM) memory, Read Only Memory (ROM) memory, an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example data may be retrieved over a modem, over the internet, or over a local area network.

The term 'computer memory' or 'memory' as used herein encompasses a computer readable storage medium which is directly accessible to a processor. Examples of computer memory include, but are not limited to: RAM memory, registers, and register files of a processor.

The term 'computer storage' as used herein encompasses any non-volatile computer readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

The term 'computing device' as used herein encompasses any device comprising a processor. The term 'processor' as used herein encompasses any electronic component which is able to execute a program or machine executable instructions. References to the computing device comprising "a processor" should be interpreted as possibly containing more than one processor. The term 'computing device' should also be interpreted to possibly refer to a collection or network of computing devices each comprising a processor. Many programs have their instructions performed by multiple processors that may be within the same computing device or which may be even distributed across multiple computing devices. The term 'computer system' may be interpreted herein as being a 'computing device.'

The term 'database' as used herein is a collection of logically-related data or files containing data that provide data for at least one use or function. Databases are essentially organized data that may be provided or used by an application. Examples of a database include, but are not limited to: a relational database, a file containing data, a folder containing individual data files, and a collection of computer files containing data.

The term 'access key' as used herein is data or a character string which is used to provide read and/or write access to a database. In some embodiments the access key may be a reference used for identifying or locating data in the database. For example, in some embodiments an access key may be a pseudonym. The pseudonym allows identification of the ownership of various records. In other embodiments an access key may be a password or user identification. In other embodiments the access key may identify a record or records within the database. Records may be individual data files or they may be a collection of data files. An access key may be a primary key for a relation in a database. An access key may also be a key for a relation in a relational database, e.g. a so-called 'secondary key', and in particular a 'primary key'.

In one aspect, the invention relates to a computer-implemented method for determining the presence of a disease in a patient. The method comprises the following steps:

- Receiving, by a computer system, first rule sets comprising rules, the rules of each first rule set being operable to evaluate a set of disease indicators, the disease indicators of each first rule set being biomedically related to each other, the rules of each first rule set being grouped into one or more second rule sets, each second rule set comprising a score value, said score value being indicative of the risk of the patient to have the disease in case the evaluation of the one or more rules of said second rule set on biomedical data of the patient returns a positive result;
- Determining, by the computer system, for each first rule set, the highest score value of its second rule sets by evaluating all rules of said second rule sets on the biomedical data of the patient;
- Calculating, by the computer system, a total score value for the disease as a derivative of the determined highest score values of all first rule sets; and
- Returning, by the computer system, a first diagnosis result, the first diagnosis result being indicative of the presence of the disease in the patient, the first diagnosis result having assigned the total score value.

According to some embodiments, the first diagnosis result is further assigned with identifiers of all first rule sets having a highest score value larger than 0. According to some embodiments, said first diagnosis result is further assigned with an identifier of the disease whose presence is to be determined in a patient. Said embodiment is advantageous, as said disease indicator allows the development of presentation tiers being specifically adapted for the particular requirements of a particular disease. As a result, a DSS covering a multitude of diseases may select the appropriate presentation tier in dependence on the disease identifier.

According to embodiments of the invention, the disease indicators comprise personal data of a patient such as age or gender, existing or past diagnoses and medication, therapies and treatments, laboratory parameters and referrals. According to some embodiments, at least some of the disease indicators are associated with a time stamp information, i.e. information on the time a particular diagnosis was made, a treatment was executed or a medication was prescribed. Said timestamp information may also be evaluated by the rules when calculating the first diagnosis result. According to preferred embodiments, at least some of the disease indicators are specified according to a medical standard such as ICD 10, LOINC or the like.

Depending on the embodiment of the invention, said method can be executed on a standalone computer system, e.g. a desktop computer or notebook residing in the practice rooms of a physician, or can be executed on a server computer operated by a 3rd party, e.g. a server operated by a medical care center. The server computer may be connected to a client computer via a network, e.g. the Internet or the intranet of a hospital. The server may receive from the client a request to execute the method, perform the method and return the calculated result to the client for further processing the result and displaying it to the user, in particular a physician.

Depending on the embodiment, the method may be implemented as one or more software modules in any programming language, e.g. Java or C#. In case the method is executed by multiple software modules, said modules may be part of an application running on a standalone computer system or may be distributed among two or more different computer system being connected to each other via a network. In some embodiments, the modules are implemented as different layers of a multi-tier software architecture. The method may also be implemented as firmware or hardware.

The first rule sets can be received from any kind of data storage such as human readable files, serialized data objects, relational databases being operatively coupled to the computer system running the method, or the like. According to preferred embodiments, the rules are stored in human readable form, in particular in the form of XML documents. This is advantageous, because said storage form allows users to add, delete and modify rules without the need to modify and/or to recompile any source code. The two or more "first set of rules" can be specified (by the operator/programmer) for a particular disease and can be identified (by the operator/programmer and the executed program) as corresponding to said disease for example by a database key shared by the rules or by the position of XML elements representing rules of the same "first set of rules" within the hierarchy of an XML tree.

According to embodiments, each rule comprises one or more conditions which are connected with each other by Boolean operators such as for example AND and OR. When a rule is evaluated by a rule engine, each condition is checked, e.g. by a comparison with a data value, thereby returning for each condition a TRUE or FALSE value as intermediate results. Depending on the Boolean operators connecting said conditions in a rule and on the intermediate result values, one TRUE or FALSE value is returned for each rule as a result. For example, a rule R1 may comprise the conditions cond1, cond2 and cond3 which are connected by Boolean operators: cond1 AND (cond2 OR cond3), whereby cond1="gender=male", cond2=icd_code_value="R1234"" and cond3="age<65". Said Rule will return a TRUE result only in case disease indicators of the patient examined, e.g. his/her age, gender or disease history expressed e.g. in ICD-10 codes, comprise the appropriate disease indicator values. In this case, R1 will return TRUE in case the patient is male and is either younger than 65 years or has been diagnosed with ICD-10 code "R1234".

"Evaluating" a set of disease indicators by a rule implies comparing at least one condition of a rule with at least one disease indicator value of the patient.

Each first rule set comprises one or more "second rule sets" which can be specified and identified e.g. by means of database keys, by their position within the hierarchy of an XML tree, or the like. Each second rule set comprises a score value.

According to embodiments of the invention, each second rule set comprises only one single rule. This is advantageous, as it makes the evaluation more transparent to a human user, e.g. the programmer or operator, compared with the use of multiple rules whose results are connected dynamically during evaluation by the program. In case one single rule per second rule set is used, all evaluated conditions and their connection to each other via Boolean operators can be easily comprehended and modified by a user without changing any source code of the rules engine/the DSS.

According to embodiments of the invention, for each first rule set, the highest score value of all second rule sets being contained in said first rule set is determined. This is done by evaluating all rules of each second rule set of said first rule set, i.e. all rules of said first rule set, on disease indicators of the patient, and obtaining a result for each of said evaluated rules. According to embodiments wherein the rules of a first rules set are rules evaluating disease indicators being particular to a specific organ system, the determined highest score value of said first rule set is indicative of the probability, that the patient has the disease when only disease indicators relating to said particular organ system are considered. Typically, said score values are indicative of a probability of having the disease in a qualitative sense, which means that the risk of having a disease is directly proportional to the highest score value of each first rule set and, correspondingly, is directly proportional to the TSV.

According to some embodiments, the majority of first rule sets may respectively correspond to a particular organ system while a minority of first rule set, e.g. one single first rule set, may correspond to a collection of rules for evaluating disease indicators which are otherwise related to each other. For example, said disease indicators of said single first rule set may evaluate a set of symptoms being related to each other, e.g. in respect to their detection method or the like.

According to embodiments, the rules of the first rule set are rules evaluating disease indicators being particular to a medical sub-discipline. Accordingly, the highest score value is in these embodiments indicative of the probability, that the patient has the disease when only disease indicators relating to that particular medical sub-discipline are considered.

Considering only the highest score value obtained for each first rules set is advantageous compared to the possibility of calculating e.g. a sum or an average: there may exist a plurality of disease indicator values affecting the risk of having a disease. According to some embodiments, the totality of conditions contained in rules of a first "second rule set" is a subset of the conditions contained in rules of a second "second rule set" of a particular first rule set. Said hierarchical organization of conditions of different second rule sets may apply to some or even the majority of second rule sets being contained in one or more first rule sets.

For example, second rule set SRS_A may comprise one rule with the conditions cond4, cond5 and cond6 while another second rule set SRS_B may comprise one rule with the conditions cond4, cond5, cond6, cond7 and cond8, whereby each condition could be a condition to be evaluated on a disease indicator value of a patient. SRS_A has assigned a score value of "60" %, SRS_B a score value of "80" %. If all disease indicators, e.g. symptoms affecting a particular organ systems, according to rule set SRS_A were observed at a particular patient, but the disease indicators corresponding to cond6-8 where not observed, evaluating the rules may return the results TRUE for SRS_A and FALSE for SRS_B. The organ type specific risk of that patient for that disease may in this case be 60%. In case all disease indicators according to rule set SRS_B would have been observed at a patient, the risk for the patient to have said disease would be 80% (still only the disease indicators of only one particular organ system would be considered at this moment).

A "total" score value (TSV) can be calculated by calculating a derivative of the determined highest score values of all first rule sets. Said "derivative score value" can be, in particular, a sum of all said determined highest score values. For example, second rule sets SRS_A and SRS_B belonging to the same first rule set may have returned the result TRUE; the highest score value of said first rule set is accordingly 80%. Another "second rule set" SRS_C belonging to another "first rule set" may comprise a score value of 10% and may have returned the result TRUE. No other rule of another "first rule set" has returned a TRUE result. The final score indicating the total risk of the patient is in this case calculated as the sum of the highest score value of each first rule set, i.e. 80%+10% (+0 for each further "first rule set") = 90%.

According to further embodiments, the total score value may be calculated e.g. by calculating the sum and normalizing the sum afterwards so that the total score value for the disease lies between 0 and 1.

The first diagnosis result comprises information on whether the patient was predicted to have a significant risk of having the disease. The first diagnosis result is assigned with the calculated total score value and may be assigned with identifiers of all first rule sets having a highest score value larger than 0. According to embodiments, the first diagnosis result is also assigned with an identifier of the disease whose presence in a patient is to be determined.

The suggested division of rules into a plurality of first and second rule sets and the calculation of a TSV as a data value being a derivative of the highest score value of each of said (e.g. organ specific) first rule sets is advantageous, because not only the total number of disease indicators has an impact on the first diagnosis result, but rather also the distribution of said disease indicators among different biomedically related categories such as organ system/medical sub-discipline or others is considered. The division of rules in first rule sets also has an impact on the content of the displayed dialogue windows and thereby also on the final score value and the second diagnosis result. The disease indicators evaluated by the rules of each first rule set are biomedically related to each other, e.g. belong to the same organ system or are examined by physicians of a particular medical sub-discipline. A result which is indicative of the totality of disease indicators positively evaluated (total score value) which takes into consideration also the distribution of said disease indicators in different organ systems is advantageous as this approach allows the definition of one or more hierarchically organized condition sets and provides for a particularly accurate diagnosis result.

According to embodiments, each first rule set corresponds to a particular 'type' of related disease indicators. The 'type' of each set of related disease indicators and, correspondingly, the type of each first rule set, thereby corresponds to the biomedical category shared by all or most of the disease indicators evaluated by the rules of a particular first rule set. Such a category can be, for example, an organ system or a biomedical sub-discipline to which at least some of the disease indicators evaluated by the rules of said first rule set relate to.

According to further embodiments of the invention, each first rule set represents a set of biomedically related disease indicators and each first rule set solely comprises rules for evaluating said biomedically related disease indicators.

According to further embodiments of the invention, each first rule set represents one organ system and each first rule set solely comprises rules for evaluating disease indicators to become manifest in said organ system.

According to further embodiments of the invention, each first rule set represents one medical sub-discipline and each first rule set solely comprises rules which comprise at least one condition on disease indicators of diseases examined in said medical sub-discipline.

According to further embodiments of the invention, the method further comprises the step of displaying the calculated first diagnosis result to the user, said displaying step comprising:

- Determining, whether the total score value exceeds a first threshold value;
- In case the total score value does exceed the first threshold value, executing the following steps:

  • Displaying the first diagnosis result in a first GUI window;
  • Displaying one or more dialog windows prompting a user to enter additional data of the patient;
  • Calculating a final score value by evaluating one or more third rule sets on the entered additional data;
  • Displaying a positive second diagnosis result in a second GUI window in case the final total score value exceeds a second threshold value, and
  • Displaying a negative second diagnosis result in a third window in case the final score value does not exceed the second threshold value.

A 'positive' result as used herein is a result, e.g. a text message, stating that the disease is present in a patient or that the patient has a high risk of having said disease, while a 'negative' result states that the disease is probably not present in said patient.

According to embodiments, the first GUI window comprises a first selectable GUI element, e.g. a button or a link, whereby one or more dialog windows are displayed to the user upon selection of said first selectable GUI element by the user.

According to embodiments, the first GUI window comprises a second selectable GUI element, e.g. a button or a link, whereby additional information on the disease is displayed to the user upon selection of said second selectable GUI element by the user, e.g. literature references relating to said disease. According to preferred embodiments, the displayed literature references depend on the disease to be determined and on the type of first rule sets having positively contributed to the TSV.

According to some embodiments, for each positively evaluated first rule set one or more of said dialog windows, also referred to as 'questionnaire dialog windows', are displayed, whereby the content of said one or more dialog windows is determined by their respective first rule set.

According to embodiments, the first diagnosis result is to be considered as "preliminary" diagnosis result. The first rule set identifiers contained therein for which a highest score value larger than zero was calculated is used by the DSS for determining which questionnaire dialog window(s) is/are to be displayed to the user. The questions displayed in said one or more questionnaire dialog windows thereby depends on the type of said first rule sets positively contributing to the TSV. According to some embodiments, for each first rule set positively contributing to the TSV value one or more questionnair dialog windows corresponding to the disease indicators evaluated by said first rule set are displayed to the user. For example, in case a first rule set relating to the renal organ system contributed to the TSV with 40%, then one or more questionnaire windows may be displayed to the user coprising questions relating to the renal system in case the TSV value exceeds a first threshold. Then, a final score value (final SV) is calculated based on data entered by the user into said one or more questionnair diag windows (for the renal organ system and all other organ system whose corresponding first rule set positively contributed to the TSV). The calculated final SV is then compared to a second threshold value. In case the final SV exceeds said second threshold value, a second diagnosis result, also called 'final diagnosis result' is displayed to the user.

The second diagnosis result comprises information on whether the patient was predicted to have the disease or not. Depending on the embodiment, a second diagnosis result may comprise the final score values and may also comprise additional information on said disease and on possible treatment options. The second diagnosis result is typically more accurate than the first diagnosis result, because disease- and organ type specific, additional data has been collected and used for calculating the final SV the second diagnosis result is based on.

According to embodiments, the method further comprises the step of loading, for each first rule set having returned a highest score value larger than 0, a third rule set. The final score value is calculated by applying the rules of said one or more third rule sets on the entered additional data.

According to embodiments, the third rule set is particular to each disease and to each set of disease indicators evaluated by a particular first rule set.

According to embodiments, the third rule sets are stored in the rules repository and evaluated by program routines in the presentation tier having access to the rules repository. According to other embodiments, the third rule sets are integral part of a presentation tier corresponding to the disease whose presence in a patient is to be determined.

According to preferred embodiments, the above steps for displaying the first and second diagnosis result are executed by a decision support system running e.g. on a computer in a medical practice or hospital. Said computer system may be a standalone computer system or a client computer system connected to a server computer system.

Depending on the embodiment, the steps of calculating the first and second diagnosis result may also be executed by some modules or tiers of a DSS on the server computer system. The DSS may be provided as remote service by a server operated by a hospital or a third party. The server computer system can be connected to one or more client computer systems via the internet, the intranet or via a laboratory information system (LIS). A client computer system may request a first and/or second diagnosis result from the remote DSS hosted on the server and may display the received diagnosis result locally.

The DSS is, according to embodiments, operatively coupled to a rule repository comprising the rules of the first and second rule sets for determining the presence of a particular disease of a patient. According to some embodiments, said rules repository in addition comprise rules of additional first and second rule sets for determining the presence of one or more additional diseases in a patient. The DSS is in addition operatively coupled to a data repository comprising disease indicators of the patient. By evaluating the rules corresponding to a particular disease, e.g. Morbus Fabry, on the disease indicators of a patient, the DSS is operable to automatically determine whether said disease is present in a patient or not. As for any other existing automated diagnosis approach, it may be recommendable in some cases to perform additional empirical examinations, e.g. laboratory tests, in order to further refine the diagnosis result. Said data repository may be an electronic health card of a patient, a local or remote relational database or any other data source. The data source may comprise solely the data of one patient or of a multitude of patients.

According to embodiments, the DSS is operable to receive a request from a client to automatically determine whether a particular disease is present in a patient or not. According to embodiments, this request comprises an identifier of the disease, thereby enabling the DSS to load the rules needed to calculate the risk for said disease from the rules repository. The request may in addition comprise an identifier of the patient, e.g. a patient-specific database access key, which enables the DSS to selectively retrieve disease indicators of said particular patient from the data repository comprising the disease indicators of one or multiple patients. Said database access key is calculated, according to some embodiments, from a pseudonym based on a user-selected secret.

According to further embodiments, the method is implemented in a DSS, whereby said DSS has a multi-tier architecture. A first tier, the data tier, provides access to the biomedical patient data. A second tier, the logic tier, comprises a rule engine and is operable to evaluate the rules of all first rule sets of a particular disease. The program logic of said logic tier for evaluating the rules on biomedical patient data is always the same irrespective of the type of loaded rules/the disease whose presence is to be determined. The first rule sets read from the rules repository may differ, however, depending on the disease to be analyzed. One or more third tiers, also referred to as 'presentation tiers', display the calculated first and second diagnosis results to a user. The number and type of dialog windows created by the third tier depends on a disease identifier of the disease whose presence is to be determined in the patient and on the type of first rule sets returning a highest score value larger than zero.

According to some embodiments, the biomedical data of the patient is collected by executing the steps:

- receiving parts of the biomedical patient data of the patient from a plurality of different data sources, whereby each received part of the biomedical patient data respectively comprises a unique identifier, the unique identifier being identical in each of the received parts of the biomedical patient data, and
- storing the received biomedical data into one database, thereby using the unique identifier as database access key for all received parts of the biomedical patient data,
- whereby the unique identifier is generated by each of the data sources by executing the steps of:

  • receiving a user-selected secret;
  • storing the user-selected secret in the first memory;
  • computing a private key by applying an embedding and randomizing function onto the secret;
  • storing the private key in the memory;
  • computing a public key by using the private key, whereby for performing the calculation of the public key a cryptographic one-way function is used, the public key and the private key forming an asymmetric cryptographic key;
  • outputting the public key for providing the access key; and erasing the secret and the private key from the memory

According to further embodiments, said method further comprises the step of accessing an input value, wherein the private key is the input value.

According to embodiments, the above method steps are executed by a server computer system and the different data sources are different client computer systems.

Said embodiments are advantageous as they provide for a database, e.g. a relational database, which comprises biomedical data of a patient having been gathered from a multitude of (potentially) independent and unconnected data sources. Typically, a patient visits a multitude of different physicians practicing in different cities and in different medical disciplines. These physicians typically do not know of each other, and accordingly the health parameters of a patient gathered by one physician are often not known to another physician. As a result, the totality of biomedical data of a patient which is theoretically available as data basis for making a prediction is in practice scattered among a multitude of different, independent practices and cannot be used to make diagnoses. As a consequence, the prediction accuracy of DSSs operating solely on parts of the theoretically available data is worse than it could theoretically be. In addition, there exists the risk that a patient is treated by different physicians with incompatible drugs and that a patient is e.g. X-rayed multiple times by different physicians unnecessarily. By gathering patient data collected by different physicians *who do no not have to know from each other,* by means of a unique and secure database access key, the biomedical data of a patient can be gathered in one common data repository, thereby increasing the data basis of DSS and improving the accuracy of the calculation results generated by said DSS.

According to preferred embodiments, the user selected secret is e.g. a passphrase and said user is the patient. The patient may visit a first physician, e.g. a cardiologist, who collects cardiological data of the patient. The cardiologist may ask the patient to enter a secret which is only known to the patient, in order to generate an access key as described above. The access key is then submitted, together with the cardiological data of the patient, to a remote, centralized biomedical database. The cardiological data of the patient is stored to said database, whereby the generated access key is used as database access key, e.g. a primary key. The same patient may later on visit an orthopedist who collects orthopedic data from the patient. The orthopedist may also ask the patient to enter his user selected secret (which has to be identical to the secret given to the cardiologist) in order to generate that access key. The orthopedic data is then also stored to the remote, centralized medical database by using that access key as database access key. Although the cardiologist and the orthopedist may not know from each other and may never exchange data with each other, the patient related data independently gathered by both specialists is stored in the same database.

According to preferred embodiments, the method steps for generating an access key from a user selected secret may be provided by a client computer. Said steps may be executed e.g. by a module of or a plug in for a doctor information system providing a user with graphical user interface means for entering a secret, e.g. a password dialogue window.

According to further embodiments, a remote, centralized medical database comprises biomedical data (e.g. disease indicators) of a plurality of patients. Biomedical data belonging to a particular patient can be identified by means of the database access key having been generated from a user selected secret as described below.

According to embodiments of the invention, the biomedical data of the patient is collected by executing the steps:

- receiving parts of the biomedical patient data of the patient from a plurality of different data sources respectively, whereby each received part of the biomedical patient data comprises a unique identifier, the unique identifier being identical in each of the received parts of the biomedical patient data, and
- storing the received biomedical data into one database, thereby using the unique identifier as database access key for all received parts of the biomedical patient data,
- whereby the unique identifier is generated by each of the data sources by executing the steps of:

  • entering a user selected secret and storing said user selected secret into a memory;
  • computing a private key by applying an embedding and randomization function onto said user selected secret;
  • storing the private key in the memory;
  • computing a public key using the private key, the public key and the private key forming an asymmetric cryptographic key pair;
  • irretrievably erasing said user selected secret and the private key from the memory after that public key is computed as said user selected secret is only known to said user and not stored on any computer; and
  • outputting the public key for providing that pseudonym, wherein that pseudonym is assigned as an identity of said user and no third party is required for establishing a binding between that pseudonym and that user's identity.

Said embodiments are advantageous because erasing said user selected secret irretrievably guarantees that it cannot be misused by any third party. The only instance knowing said user selected secret is the user himself, typically the patient.

According to other embodiments, the outputted pseudonym is used as an identifier for the user/the patient in a network in addition to or alternatively to using said pseudonym as database access key. For example, the user may be allowed to access his biomedical data stored to a centralized data repository via a HTML interface, whereby the pseudonym is used as a username e.g. during the login process and/or for identifying the user within the network.

Depending on the embodiment, each client computer system may store biomedical data of a patient in the remote centralized database directly, e.g. via a database connection established e.g. by a doctor information system, or indirectly by submitting the biomedical data and the access key to a remote service having access to said centralized database.

According to embodiments of the invention, the method for returning a diagnosis of a patient is implemented by a DSS being operatively coupled to a database. The database comprises biomedical data of the patient. The rules of each first rule set are evaluated on said biomedical data at an event being selected from the group consisting of:

- the moment of starting the doctor information system;
- the moment of accessing the biomedical patient data of the patient; and
- at a predefined time and date.

According to some embodiments, the DSS provides an interface to a doctor information system and the execution of the rules engine evaluating the rules for one or more diseases can be triggered by a user action executed by a user while working with said doctor information system, e.g. opening the electronic patient record of a patient. These embodiments are advantageous, because a physician does not have to initiate the evaluation of rules manually in a separate program, but rather can work with the doctor information system as he is used to. In addition embodiments of the invention allow evaluating all rules corresponding to one or more particular diseases on biomedical data of a plurality of patients, e.g. via said interface of the doctor information system. Depending on the size of the biomedical data sets and on the number of rules in the rules repository, such a 'batch run' may take several minutes or hours. Executing the evaluation in the form of a scheduled job at a predefined time and date is advantageous because it can be executed at night or at any time when the computer system is not used for entering patient related data or is performing interactive tasks.

According to further embodiments, evaluating the one or more rules of each first rule set further comprises the steps of:

- after having evaluated the rules, storing information indicating that the rules have been evaluated;
- before evaluating the rules, checking whether said information has already been stored and evaluating the rules only in case said information has not yet been stored.

Said embodiments are advantageous, because it is ensured that the rules are only evaluated if necessary when new data is available, thereby saving processing resources. According to some embodiments, said information may be deleted in case new data being indicative of a new symptom or disease of the patient is entered, e.g. directly to the DSS or indirectly via a doctor information system being interoperable with said DSS.

According to further embodiments, the rules of the first rule sets are received by a DSS from a rules repository, whereby the rules are stored in the rules repository in a data format allowing to modify or exchange the rules without introducing changes to the decision support system or the structure of the rules repository. According to preferred embodiments, said data format is an XML file format or a record in a relational database.

According to some embodiments, the rules of the first rule sets are received by a decision support system together with the first threshold value. This has the advantage that the rule engine evaluating a rule can act highly generically and flexibly at the same time. The operator of a system may adapt a first threshold value by simply editing the rules repository (e.g. a file or a database), thereby increasing or decreasing the accuracy of the prediction method. It is not necessary to introduce any changes to the source code of the rule engine. According to embodiments, the second threshold to be compared with the final score value is encoded in the presentation tier being particular to the disease in question.

In a further aspect, the invention relates to a computer readable storage medium having stored therein instructions, which when executed by a computing device, cause the computing device to perform the steps of the method for determining whether a disease is present in a patient as described above. Depending on the embodiment, said computer readable storage medium may e.g. be a hard drive or other form of storage medium of a client- or server- computer. In the case of a distributed server-client system, wherein the DSS is hosted on the server computer system and the first and second diagnosis results are displayed to a user on the client computer system, said computer readable storage medium may also comprise multiple storage media.

In a further aspect, the invention relates to a computer system comprising:

- a first memory,
- a database,
- a rules repository, and
- a processor. The processor is operatively coupled to the rules repository and the database. The processor is operable to execute instructions stored in the memory, whereby the memory contains instructions for performing the steps of the method for determining whether a disease is present in a patient as described above. The rules are received from the rules repository. The database comprises the biomedical data of the patient. Depending on the embodiments, said computer system can be a client-or a server computer system. A processor being 'operatively coupled' to a rules repository and a database is a processor being operable to execute read and/or write operations on said database, the operations depending on the rules having been read from the rules repository. For example, if a rule comprises a condition regarding the age of a patient, the disease indicator value "age" of said patient is read from the database and compared by the processor with a numerical value specified in said condition.

According to some embodiments, said computer system further comprises a user interface for entering a user-selected secret, whereby the first memory or a second memory contains instructions for performing the steps of:

- receiving a user-selected secret;
- storing the user-selected secret in the memory ;
- computing a private key by applying an embedding and randomizing function onto the secret;
- storing the private key in the memory;
- computing a public key by using the private key, whereby for performing the calculation of the public key a cryptographic one-way function is used, the public key and the private key forming an asymmetric cryptographic key;
- outputting the public key for providing an access key; and
- erasing the secret and the private key from the memory.

Preferentially, said computer system is a client computer system comprising a DSS. Preferentially, said client computer system is also operable to store biomedical data of a patient into a local or a remote, centralized database by using said access key as database access key.

In a further aspect, the invention relates to a computer system comprising:

- a database,
- one or more first computing devices, each first computing device being operatively coupled to the database and comprising:

  • a processor,
  • a memory, wherein the memory contains instructions for performing steps for generating a pseudonym, said steps comprising:

- ∘ receiving a user-selected secret;
- ∘ storing the user-selected secret in the memory;
- ∘ computing a private key by applying an embedding and randomizing function onto the secret;
- ∘ storing the private key in the memory, wherein the private key is the input value;
- ∘ computing a public key using the private key by using the cryptographic one-way function, the public key and the private key forming an asymmetric cryptographic key;
- ∘ outputting the public key for providing an access key; and
- ∘ erasing the secret and the private key from the memory.

- • means for storing biomedical data into the database, whereby the public key is used as database access key for storing biomedical data of the user into the database,

whereby the stored biomedical data in the database constitutes the data basis for a decision support system being operable to automatically determine whether one or more orphan diseases is present in the user.

According to preferred embodiments, said one or more computing devices are client computer systems.
In a further aspect, the invention relates to a computing system comprising:

- - a database,
- - one or more first computing devices, each first computing device being operatively coupled to the database and comprising:

- • a processor,
- • a first memory, wherein the memory contains instructions for performing steps for generating a pseudonym, said steps comprising:

- ∘ receiving a user-selected secret;
- ∘ storing the user-selected secret in the memory;
- ∘ computing a private key by applying an embedding and randomizing function onto the secret;
- ∘ storing the private key in the memory;
- ∘ computing a public key by using the private key, whereby for performing the calculation of the public key a cryptographic one-way function is used, the public key and the private key forming an asymmetric cryptographic key;
- ∘ outputting the public key for providing an access key; and

- • erasing the secret and the private key from the memorymeans for storing biomedical data into the database, whereby the public key is used as database access key for storing the biomedical data of the user into the database,

- ∘ whereby the stored biomedical data in the database constitutes the data basis for a decision support system,
- ∘ whereby said biomedical data of the patient comprises disease indicator values belonging to two or more groups of biomedically related disease indicator groups,
- ∘ whereby said decision support system is operable to determine the presence of a disease in the patient by executing instructions, said instructions implementing steps comprising:

- - Determining, for each group of disease indicators, a score value, said score value being indicative of the risk of the patient to have the disease when solely the disease indicators of said group are evaluated,
- - Calculating a total score value for the disease as a derivative of the determined score values of all disease indicator groups;
- - Returning a first diagnosis result, the diagnosis result being indicative of the presence of the disease in the patient, the diagnosis result being assigned with the total score value;

According to preferred embodiments, said one or more computing devices are client computer systems.
Providing a pseudonym for a user is as such known. A pseudonym is typically used for protecting the informational privacy of a user such as in a social network. Such computer implemented schemes for providing a pseudonym typically enable the disclosure of identities of anonymous users if an authority requests it, if certain conditions are fulfilled.

For example, Benjumea et al, Internet Research, Volume 16, No. 2, 2006 pages 120-139 devise a cryptographic protocol for anonymously accessing services offered on the web whereby such anonymous accesses can be disclosed or traced under certain conditions.

Some embodiments of the present invention are particularly advantageous as an extremely high degree of protection of the informational privacy of users is provided. This is particularly advantageous when sensitive, medical patient data is transmitted via a network to be stored in or retrieved from a central data repository. The high security level is due to the fact that an assignment of the user's identity to the user's pseudonym does not need to be stored and that no third party is required for establishing a binding between the pseudonym and the user's identity. In contrast, some embodiments of the present invention enable to generate a user's pseudonym in response to the user's entry of a user-selected secret whereby the pseudonym is derived from the user-selected secret. As the user-selected secret is known only by the user and not stored on any computer system there is no feasible way that a third party could break the informational privacy of the user, even if the computer system would be confiscated such as by a government authority.

This enables to store sensitive user data, such as medical data, in an unencrypted form in a publicly accessible database. The user's pseudonym can be used as a database access key, e.g. a primary key or candidate key value that uniquely identifies tuples in a database relation, for read and write access to data objects stored in the database.

For example, the database with pseudonymous data can be used for a decision support system, e.g. in the medical field for evaluating a user's individual medical data and processing the data by rules. The result of the evaluation and processing by rules may be hints and recommendations to the physician regarding the user's health condition and further treatment.

The invention provides for a computer readable storage medium having stored therein instructions. When the instructions are executed by a computing device the instructions cause the computing device to perform a method of generating an access key. The method comprises the step of accessing an input value. The method further comprises the step of calculating an asymmetric cryptographic key pair by applying a cryptographic one-way function to the input value. The cryptographic key pair comprises a public key and a private key. The cryptographic one-way function is an injective function. The method further comprises the step of outputting the public key for providing the access key. Essentially the public key is the access key. This embodiment is advantageous because the input value may be used to generate the access key. A user operating the computing device therefore does not need to know the access key. The user can obtain the access key by executing the instructions on the computing device.

In another embodiment the method further comprises the step of depositing data into a database using the access key. This embodiment is advantageous because the access key may be used to control access or control data that is able to be written into the database. Alternatively the access key could be used as a pseudonym for which data deposited into the database is referenced against. This provides anonymity for a user.

In another embodiment the method further comprises the step of generating a digital signature for the data using the private key. The digital signature is deposited into the database, associated with the data. This embodiment is particularly advantageous because the digital signature for the data allows authentication of the data. In this way the authorship of the data can be verified.

In another embodiment the method comprises the step of verifying the authenticity of the data using the access key. This embodiment is advantageous because the authenticity or authorship of the data can be verified using the access key.

In another embodiment the access key is used as a pseudonym by the database. In this embodiment the data which was deposited into the database is referenced as being deposited by a specific person or entity using a pseudonym. An advantage of this embodiment of the method is that data can be stored or referenced in a database using the pseudonym without revealing the identity of who placed the data into the database.

In another embodiment the input value is the private key. This is advantageous because the input or private key may be stored within the computer readable storage medium or another computer storage medium and kept securely. The private key can then be used to generate a unique access key for the database.

In another embodiment the method further comprises the step of calculating a first public key using the input value and a first base point. The public key is calculated using asymmetric cryptography which is implemented using elliptical curve cryptography. The method further comprises the step of outputting the first public key as a pseudonym. This embodiment is advantageous because a private key has been used to generate a pseudonym calculated from a public key using elliptic curve cryptography. A pseudonym has been generated for which the input value or private key cannot be inferred.

In another embodiment the method further comprises the step of calculating a second public key using the input value and a second base point. The second base point is different from the first base point and cannot be inferred from it. The method further comprises the step of outputting the second public key as a public key for the encryption of data. This embodiment is advantageous because a single input value or private key has been used to generate both a pseudonym and a public key for the encryption of data. This is particularly advantageous because both values cannot be inferred from each other, yet only a single input value is needed for both. In other words, knowledge of one of the base points does not allow an attacker to determine the other base point. The two base points are therefore not correlatable. However, both of the base points are determined by a single input value or private key.

In another embodiment the cryptographic one-way function comprises an embedding and/or randomizing function. This is advantageous because the input value may be clear text or an easily guessed value. By using an embedding and/or randomizing function a pseudonym which is more difficult to decrypt may be constructed.

In accordance with an embodiment of the invention, at least one public parameter is used for applying the embedding and randomization function. A public parameter may be the name of the user, an email address of the user or another identifier of the user that is publicly known or accessible. A combination of the user-selected secret and the public parameter is generated by the embedding component of the embedding and randomization function that is applied on the user-selected secret and the public parameter.

The combination can be generated such as by concatenating the user-selected secret and the public parameter or by performing a bitwise XOR operation on the user-selected secret and the public parameter. This is particularly advantageous as two users may by chance select the same secret and still obtain different pseudonyms as the combinations of the user-selected secrets with the user-specific public parameters differ.

In accordance with an embodiment of the invention, the embedding component of the embedding and randomizing function comprises a binary cantor pairing function. The user-selected secret and the public parameter are embedded by applying the binary cantor pairing function on them.

In accordance with an embodiment of the invention, the randomizing component of the embedding and randomizing function uses a symmetric cryptographic algorithm like the Advanced Encryption Standard (AES) or the Data Encryption Standard (DES) by means of a symmetric key. This can be performed by encrypting the output of the embedding component of the embedding and randomizing function, e.g. the binary cantor pairing function, using AES or DES.

In accordance with an embodiment of the invention, the symmetric key that is used for randomization by means of a symmetric cryptographic algorithm is user-specific. If the symmetric key is user-specific, the use of a public parameter can be skipped, as well as embedding the user-selected secret and the public parameter; the randomizing function can be applied then solely on the user-selected secret. By applying a symmetric cryptographic algorithm onto the user-selected secret using a user-specific symmetric key both embedding and randomization of the user-selected secret are accomplished. If the symmetric key is not user-specific, the use of the public parameter and embedding the user-selected secret and the public parameter are necessary.

In accordance with an embodiment of the invention, the embedding and randomizing function is implemented by performing the steps of applying a first one-way function on the user-selected secret to provide a first value, providing a random number, embedding the random number and the first value to provide a combination, and applying a second one-way function on the combination to provide a second value, wherein the second value constitutes the private key. This embodiment is particularly advantageous as it provides a computationally efficient method of implementing an embedding and randomization function.

In accordance with an embodiment of the invention, the computation of the public key is performed by elliptic curve cryptography (ECC). The private key that is output by the embedding and randomizing function is multiplied with a first base point given by the domain parameters of an elliptic curve to provide another point on the elliptic curve, which is the pseudonym.

In accordance with an embodiment of the invention, it is determined whether the output of the embedding and randomizing function fulfils a given criterion. For example, it is checked whether the output of the embedding and randomization function is within the interval between 2 and n-1, where n is the order of the elliptic curve. If the output of the embedding and randomizing function does not fulfill this criterion another random number is generated and the embedding and randomization function is applied again to provide another output which is again checked against this criterion. This process is performed repeatedly until the embedding and randomizing function provides an output that fulfils the criterion. This output is then regarded as the private key that is used to calculate the public key, i.e. the pseudonym, by multiplying the private key with the first base point.

In accordance with a further embodiment of the invention the base point is varied leaving the other domain parameters unchanged for computation of multiple pseudonyms for a given user. This provides a computationally efficient way to compute multiple pseudonyms for a given user in a secure way.

In another aspect the present invention relates to a computer readable storage medium having stored therein instructions, which when executed by a computer system, cause the computer system to generate a pseudonym for a user upon a user's entry of a user-selected secret by performing the steps of storing the user-selected secret in memory, computing a private key by applying an embedding and randomizing function onto the secret and possibly additional public parameters, storing the private key in memory, computing a public key using the private key, the public key and the private key forming an asymmetric cryptographic key pair, erasing the secret and the private key from memory, outputting the public key for providing the pseudonym.

In another aspect the present invention relates to a computer system comprising means for entering a user-selected secret, memory means for storing the user-selected secret and a private key, processor means being operable to compute the private key by applying an embedding and randomizing function onto the secret and possibly additional public parameters, compute a public key using the private key, the public key and the private key forming an asymmetric cryptographic key pair, erase the secret and the private key as well as any intermediate computational results from memory, and output the public key for providing the pseudonym.

In another aspect the invention provides for a computer implemented method of generating an access key. The method comprises the step of accessing an input value. The method further comprises the step of calculating an asymmetric cryptographic key pair by applying a cryptographic one-way function to the input value and further steps. The cryptographic key pair comprises a public key and a private key. The cryptographic one-way function is an injective function. The method further comprises the step of outputting the public key for providing the access key. This embodiment is advantageous because the input value is used to calculate a pseudonym using a cryptographic one-way function. In some embodiments, the access key can be used by a user as a pseudonym for many different situations for instance for an online forum or in order to keep medical records private. The advantage of using an input value to generate a pseudonym e.g. by using a cryptographic one-way function is that it is not necessary to store a table with users and their pseudonyms. This increases the security of the pseudonym because the input value can be kept private and not shared or stored within a system. Because the pseudonym is calculated using a cryptographic one-way function the input value will be impossible to calculate from the pseudonym.

In another aspect the invention provides for a computing device comprising a processor and a memory. The memory contains instructions for performing a method of generating an access key. The method comprises the step of accessing an input value. The method further comprises the step of calculating an asymmetric cryptographic key pair by applying a cryptographic one-way function to the input value and further steps. The cryptographic key pair comprises a public key and a private key. The cryptographic one-way function is an injective function. The method further comprises the step of outputting the public key for providing the access key. The advantages of the method performed by executing the instructions have been previously discussed.

In another embodiment the computing device is any one of a cellular telephone, a smart card, a security token, a personal digital system, an RFID tag, an RFID card, a computer, and a computer system. In the case of security token the computing device may also comprise components or a computer external to the security token. For instance if the security token simply has storage for the input value, then the computing device may be a computer or other computing device which accesses the memory of the security token. The computing device may be a computer system.

In another embodiment the input value is a private key which can be used for calculating at least one public key to form at least one asymmetric cryptographic key pair. The advantages of this embodiment have been previously discussed.

In another embodiment the computing device comprises memory wherein the input value is stored. In this embodiment the input value is stored within the memory and is accessible by reading the memory from the computing device. In this case the input value may be secured by securing the computing device. For instance in the case of a smart card or an RFID card the input value may be stored in secure memory which may not be accessed without proper access instructions and which is physically protected from tampering.

In another embodiment a user-selected secret is received from a user interface. The input value is derived from the user-selected secret. In this embodiment security for the input value is provided by not storing it in the computing device. The input value is generated from a user-selected secret.

In another embodiment the computing device comprises a user interface for entering a user-selected secret. The computing device further comprises a memory for storing the user-selected secret and a private key. The computing device further comprises a processor operable for executing instructions stored in the memory. The memory contains instructions for performing the step of receiving a user-selected secret. The memory further comprises instructions for performing the step of storing the user-selected secret in memory. The memory further contains instructions for performing the step of computing a private key by applying an embedding and randomizing function onto the secret and possibly additional public parameters. The memory further contains instructions for performing the step of storing the private key in the memory. The private key is the input value. The memory further contains instructions for performing the step of computing a public key using the private key using a cryptographic one-way function. The public key and the private key form an asymmetric cryptographic key pair. The memory further contains instructions for performing the step of outputting the public key for providing the pseudonym. The memory further contains instructions for performing the step of erasing the secret and the private key from the memory.

**Brief description of the drawings**

In the following, embodiments of the invention are explained in greater detail, by way of example only, making reference to the drawings in which:

Figure 1a     is a flowchart of a method for automatically determining the presence of a disease in a patient,

Figure 1b     is a flowchart for displaying first and seconddiagnosis results,

Figure 2     is a block diagram of a decision support system comprising multiple tiers,

Figure 3a     is a block diagram of a client computer system comprising a doctor information system and a DSS,

Figure 3b     is a block diagram showing a client computer system and a server computer system connected to each other via a network,

Figure 4a     comprises screenshots of three variants of a window with a positive first diagnosis result,

Figure 4b     shows disease pattern specific questionnaires for calculating a final score value,

Figure 4c     displays a negative result window and a positive result window comprising a second diagnosis result,

Figure 5     is a block diagram of a first embodiment of a computer system of the invention,

Figure 6     is a flowchart being illustrative of an embodiment of a method of the invention for providing a database access key,

Figure 7     is a block diagram of a further embodiment of a computer system of the invention,

Figure 8     is a flowchart being illustrative of a further embodiment of a method of the invention for providing a database access key,

Figure 9     is a flowchart being illustrative of a further embodiment of a method of the invention for providing a database access key,

Figure 10     is a flowchart being illustrative of a further embodiment of a method of the invention,

Figure 11     is a block diagram of an embodiment of the invention of a computing device implemented as a cellular telephone,

Figure 12     is a block diagram of a further embodiment of the invention of a computing device implemented as a security token, and

Figure 13     is a block diagram of a further embodiment of the invention of a computing device implemented as a smart card.

## Detailed description

Throughout the following detailed description like elements of the various embodiments are designated by identical reference numerals.

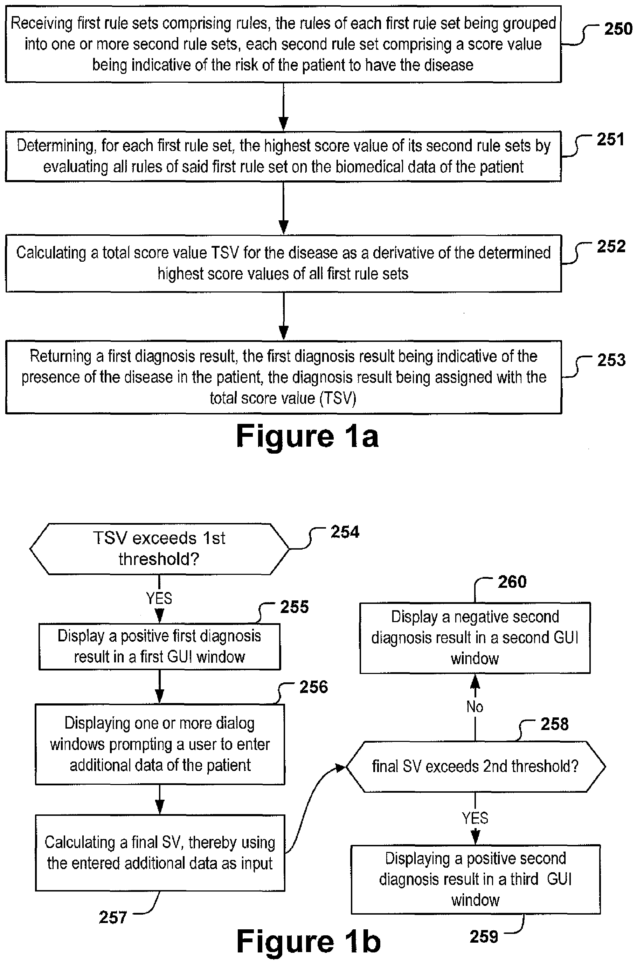

**Figure 1a** is a flowchart of a method comprising the steps of receiving 250 first rule sets comprising rules, the rules of each first rule set being operable to evaluate a set of disease indicators, the disease indicators of each first rule set being biomedically related to each other. The rules of each first rules set are grouped into one or more second rule sets. Each second rule set comprises a score value, said score value being indicative of the risk of the patient to have the disease in case the evaluation of the one or more rules of said second rule set on biomedical data of the patient return a positive result. For example, said disease can be an orphan disease, for example Morbus Fabry.

In table 1, seven different first rule sets (RS I-VII) for determining the presence of Morbus Fabry are given, whereby each first rule set corresponds to an organ system or 'disease pattern' such as 'pain-codes', i.e. a set of biomedically related disease indicators (e.g. symptoms, diagnostic codes or the like). As said disease patterns are typically related to particular organ systems, they will in the following be subsumed under the expression 'organ system'.

Each first rule set comprises one or more second rule sets. For example, first rule set RS I comprises the second rule sets RS 1, RS 8, RS 15, RS 22 and RS 29. First rule set RS II comprises the second rule sets RS 2, RS 9, RS 16, RS 23 and RS 30. Each second rule set comprises/is associated with a score value shown in table 1 in the first column. According to the depicted embodiment, only five particular score values (80%, 40%, 30%, 20% and 10%) are defined. In case the rules of a particular second rule set are evaluated on biomedical data of a patient and return a positive result, which means that the disease indicator(s) of the patient meet the condition(s) of the rule(s) of said second rule set, the score value of the first column in the respective table row is considered as result for said second rule set. If the disease indicators of the patient do not meet the condition(s) of said second rule set, "0%" is returned as result of said second rule set.

Each second rule set may comprise one or multiple rules. The rules used for determining the presence of Morbus Fabry in a patient are depicted in tables 2-8 in greater detail for each organ system respectively. According to the depicted embodiment, at least some rules comprise inclusion criteria as well as exclusion criteria. The terms "criterion" and "condition" are used herein synonymously. The presence of an exclusion criterion in a patient implies that the corresponding rule/second rule set will return as its score value the value 0 irrespective of the result obtained for the other conditions of said rule.

**Table 1**

| Score | 1st RS I<br>Pain-codes | 1st RS II<br>Nephrology | 1st RS III<br>Cardiology | 1st RS IV<br>Neurology | 1st RS V<br>Other organ systems | 1st RS VI<br>General Symptoms | 1st RS VII<br>Differential Diagnostics |
|---|---|---|---|---|---|---|---|
| 80% | 2nd RS 1 | 2nd RS 2 | 2nd RS 3 | 2nd RS 4 | - | - | - |
| 40% | 2nd RS 8 | 2nd RS 9 | 2nd RS 10 | 2nd RS 11 | - | - | - |
| 30% | 2nd RS 15 | 2nd RS 16 | 2nd RS 17 | 2nd RS 18 | - | - | - |
| 20% | 2nd RS 22 | 2nd RS 23 | 2nd RS 24 | 2nd RS 25 | 2nd RS 26 | 2nd RS 27 | 2nd RS 28 |
| 10% | 2nd RS 29 | 2nd RS 30 | - | 2nd RS 32 | 2nd RS 33 | 2nd RS 34 | 2ndRS35 |

**Table 2**

| | inclusion criteria/conditions | exclusion criteria/conditions |
|---|---|---|
| **2nd RS 1 (80%)** | Set of ICD 10 codes + pain therapy + gender:m + age:10a=<age>=20a | Trauma, rheumatism, arthritis, cancer |
| **2nd RS 8 (40%)** | Set of ICD 10 codes + gender:m + age: 10a=<age>=20a | Trauma, rheumatism, arthritis, cancer |
| **2nd RS 15 (30%)** | Set of ICD 10 codes + gender:m + age: 20a=<age>=30a | Trauma, rheumatism, arthritis, cancer |
| **2nd RS 22 (20%)** | set of ICD 10 codes | Trauma |
| **2nd RS 29 (10%)** | Set of ICD 10 codes and/or pain therapy in 2 different years | |

**Table 3**

| | inclusion criteria/conditions | exclusion criteria/conditions |
|---|---|---|
| **2nd RS 2 (80%)** | End-stage renal failure, age<40a | Diabetes, hypertensive renal disease; hypertension before renal failure |
| **2nd RS 9 (40%)** | Set of ICD 10 codes and age < 40a | Diabetes, hypertensive renal disease; |
| **2nd RS 16 (30%)** | Set of ICD 10 codes | Diabetes, hypertensive renal disease; |
| **2nd RS 23 (20%)** | Proteinuria,(gender:m and age<40a) or (gender=f, age<=50a) | - |

(continued)

| | inclusion criteria/conditions | exclusion criteria/conditions |
|---|---|---|
| 2nd RS 30 (10%) | Set of ICD 10 codes | - |

**Table 4**

| | inclusion criteria/conditions | exclusion criteria/conditions |
|---|---|---|
| 2nd RS 3 (80%) | KHK and age= <38a | Massive metabolic syndrome, smoking, heretitary dyslipidaemia, diabetes, congenital heart malformation, valve damage,atherosclerosis |
| 2nd RS 10 (40%) | Left ventricular hypertrophy | Hypertension before LH, hypertensive heart disease, heart valve disease, aorta stenosis, alcoholism |
| 2nd RS 17 (30%) | Set of ICD 10 codes | Smoker, hypertension, diabetes, alcoholism |
| 2nd RS 24 (20%) | Set of ICD 10 codes | - |
| 2nd RS 31 (10%) | - | - |

**Table 5**

| | inclusion criteria/conditions | exclusion criteria/conditions |
|---|---|---|
| 2nd RS 4 (80%) | Set of ICD 10 codes + ((gender: m: + age=<40) or (gender:w + age=<45)); | Smoker, Diabetes,Hypertension; |
| 2nd RS 11 (40%) | Set of ICD 10 codes and age <40a, w: Alter=<45; | Smoker Diabetes |
| 2nd RS 18 (30%) | ICD 10 code from list + ((gender:m + age= <40) or (gender:w+age=<45)) | - |
| 2nd RS 25 (20%) | Hypakusis+ Tinnitus | Trauma, damage caused by infection |
| 2nd RS 32 (10%) | Apoplex, TIA | - |

**Table 6**

| | inclusion criteria/conditions | exclusion criteria/conditions |
|---|---|---|
| 2nd RS 5 | - | - |
| 2nd RS 12 | - | - |
| 2nd RS 19 (30%) | | - |
| 2nd RS 26 | 2 and more different organ systems from list | - |
| | (ICD 10/ATC) are affected + age <=40a- | |
| 2nd RS 33 (10%) | Another organ system from list (ICD 10/ATC) is affected | - |

**Table 7**

|  | inclusion criteria/conditions | exclusion criteria/conditions |
|---|---|---|
| 2nd RS 6 | - | - |
| 2nd RS 13 | - | - |
| 2nd RS 20 (30%) | - | - |
| 2nd RS 27 (20%) | (ICD 10 code list or condition list) + age=<30a | - |
| 2nd RS 34 | ICD 10 code list or condition list | - |

**Table 8**

|  | inclusion criteria/conditions | exclusion criteria/conditions |
|---|---|---|
| 2nd RS 7 | - | - |
| 2nd RS 14 | - | - |
| 2nd RS 21 | - | - |
| 2nd RS 28 (20%) | Set of ICD 10 codes with status=suspicion or status=exclusion | - |
| 2nd RS 35 (10%) | Set of ICD 10 codes with status=proven | - |

The expression 'set of ICD 10 codes/ICD 10 code list' is indicative of sets of (here not further specified) ICD 10 codes. In case said codes are contained in the biomedical data of a patient, the corresponding condition returns TRUE. Then, for each first rule set (for each organ system/for each column), the highest score value of all second rule sets contained therein is determined 251 by evaluating all rules of said second rule sets on the biomedical data of the patient. For example, when the second rule sets of the renal organ system are evaluated and in case the second rule sets 2 and 9 have been evaluated positively with a score value of 40% and 80% respectively, then only the score value 80% is returned as the result obtained for said particular first rule set/renal organ system. Only the highest score value of all second rule sets having been positively evaluated on the biomedical patient data is returned as a result for each particular first rule set.

A total score value TSV is calculated 252 for a disease as a derivative of the determined highest score values of each respective first rule sets. For example, in case the highest score value obtained for the first rule set I (pain codes) was 40%, for the first rule set II (renal organ system) was also 40% and for the first rule set III (cardiological organ system) was 10%, then the total score value TSV may be calculated as the sum of all organ system specific score values, which is in this case 90%. As it is possible that said calculated total score value exceeds 100%, according to some embodiments a normalization step is executed. Then, a first diagnosis result being assigned with the total score value is returned. Said first diagnosis result may also be assigned with the identifiers of all first rule sets having a highest score value larger than 0.

According to embodiments, by forwarding the first diagnosis result having been generated in the logic tier to the presentation tier, information on the type of first rule set positively contributing to the TSV is forwarded, thereby allowing to dynamically adapt the dialog windows in dependence on the first rule sets positively contributing to the TSV. "Positively contributing" implies that the score value calculated for a particular first rule set is larger than zero.

A first diagnosis result which reflects the total number of matching disease indicators as well as their distribution among different organ system is advantageous, because the presence of a disease which has been found to be manifest in multiple organ systems at the same time in a patient can be considered as having been predicted with a particularly high accuracy. In addition, a diagnosis result being indicative of the organ system(s) wherein disease indicators have been observed, can be used as input for other program modules or programs which, based on said information, gather additional information (interactively from the patient or from a database) which is particular for said organ system in the context of the disease whose presence is to be determined. This is advantageous, because displaying questionnair dialog windows for all possible organ systems would confuse the user. By dynamically determining e.g. the type of affected organ systems and by selectively displaying dialog windows comprising questions specifically directed to said diseases, a user can very quickly and efficiently enter additional relevant patient data and can receive an according highly accurate second diagnosis result.

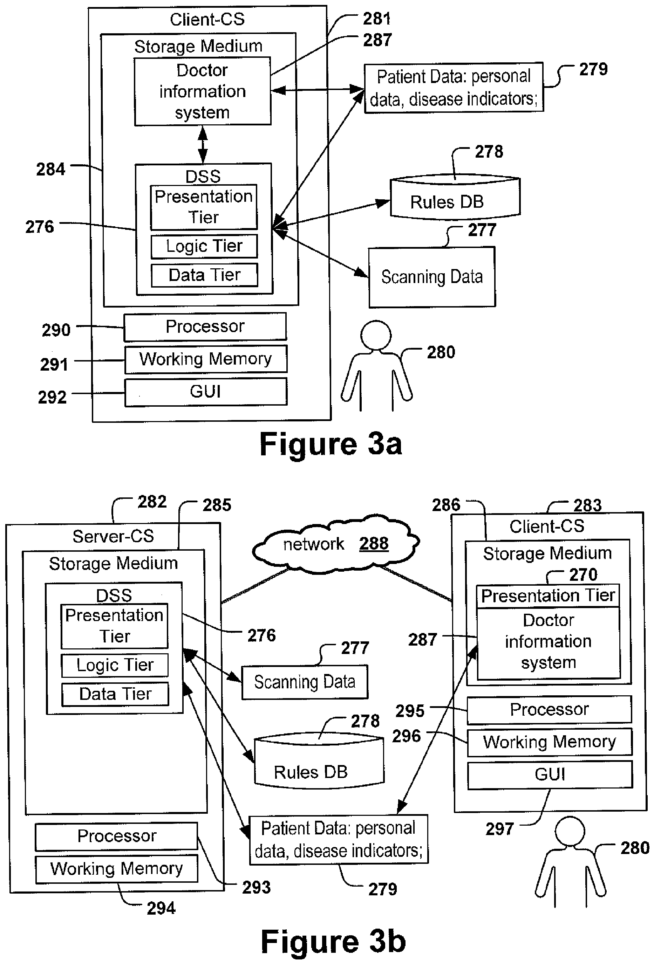

The method described in figure 1a can be executed e.g. on a standalone computer system 281 as depicted in figure 3a in greater detail. The methods may also be executed by a server computer system 282 which may return the first and second diagnosis results via a network 288 to a client computer system 283 as depicted in figure 3b. According to preferred embodiments, the method is executed by a rule engine of a decision support system. According to embodiments, a second diagnosis result comprises a numerical probability value or textual expression being indicative of the probability of the patient for having the disease and may in addition comprise additional information on the disease e.g. in the form of links to additional information sources.

According to some embodiments, a user, typically a physician, may choose between executing the method depicted in figure 1 a once for one particular patient and one particular disease, e.g. Morbus Fabry, and executing said method for a particular disease for *all* patients whose data are available. According to some embodiments, the user may also apply a multitude of first rule sets relating to a multitude of different diseases on biomedical data of one or multiple patients.

According to preferred embodiments, the evaluation of the rules of the first and second rule sets for more than one patient at the same time is executed as a batch job at a moment in time when said batch job does not use up processing resources required by user-interactive jobs. Executing batch jobs for a multitude of diseases on a multitude of patients has the advantage that a disease may be detected even in case the patient has not visited the physician for a long time. It may be possible that new rules based on new biomedical knowledge is used by a decision support system for disease prediction and may therefore be able to detect a disease which was not detected some years ago at the last doctor's visit of the patient. The physician may notify the patient on the diagnosis result and treatment options.

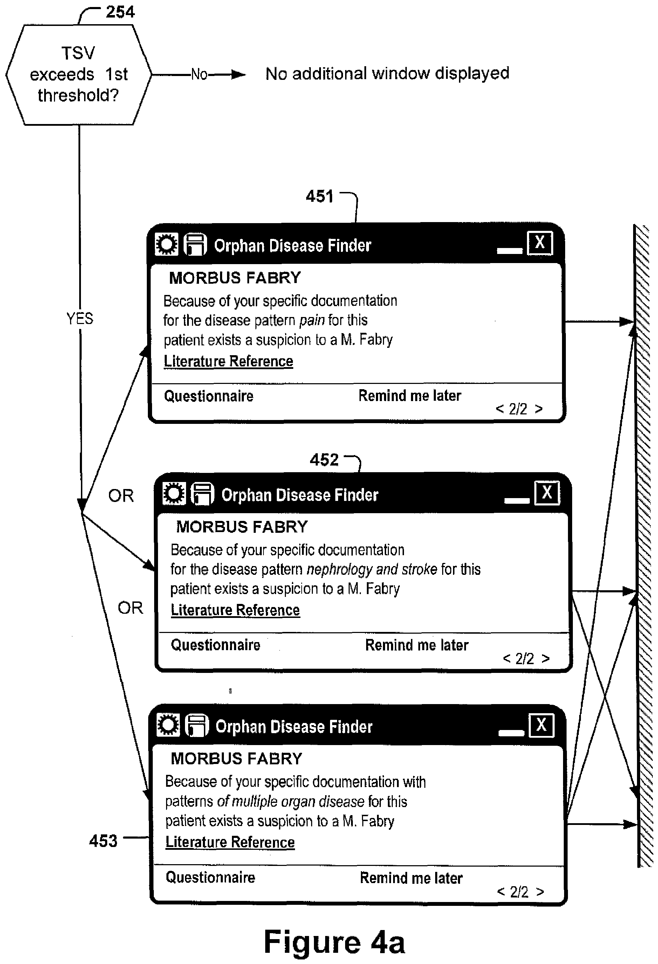

**Figure 1b** shows a flow flowchart comprising steps for displaying a first and second diagnosis result. In a first decision, it is decided 254, whether the TSV exceeds a first threshold value or not. The first threshold value may be predefined e.g. in configuration data or the source code of the decision support system executing the methods depicted in figure 1a. According to preferred embodiments, the first threshold is received together with the first rule sets for a particular disease i.e., it is a disease specific first threshold that can easily be edited in the rules repository. In case it is found that that TSV exceeds this first threshold, a positive first diagnosis result is displayed 255 in a first GUI window 451, 452, 453 depicted in fig. 4a. According to embodiments, the text message displayed in said first GUI windows is indicative of all first rule sets (or their respective organ systems, medical sub-disciplines or the like) positively contributing to the TSV. For example, first GUI window 451 comprises the text string 'for the disease pattern pain' while an alternative version 452 of the first GUI window comprises the text string 'for the disease pattern nephrology and stroke'. In case the total score value does not exceed said first threshold, no message is displayed to the user to avoid disturbing the user with irrelevant messages.

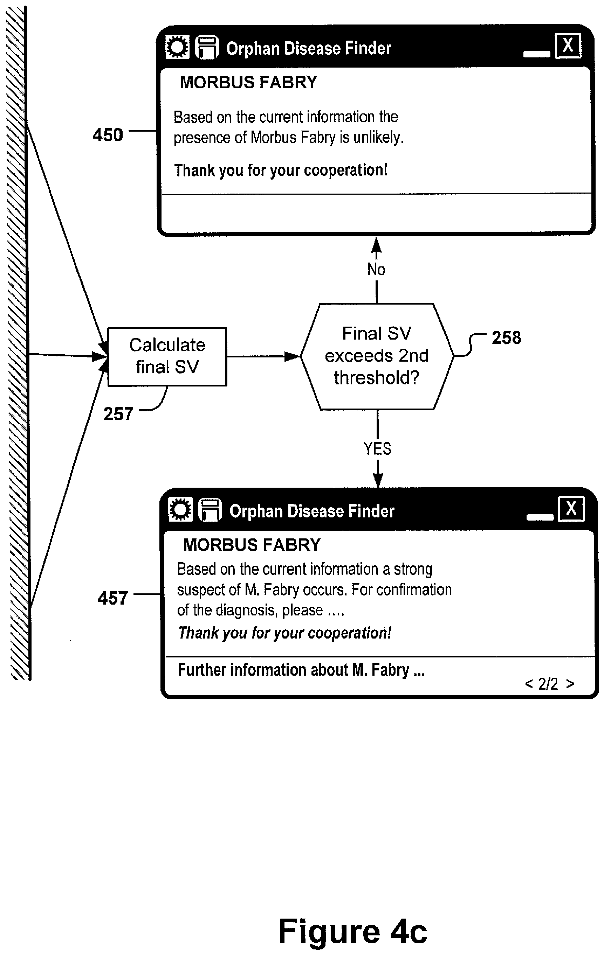

In a further step 256, one or more dialog windows 454, 455, 456, also referred to as 'electronic questionnaires' or 'questionnair dialog windows', prompting a user to enter additional data of the patient are displayed. Depending on the embodiments, said step 256 may be executed automatically or upon a selection of a GUI element, e.g. a link being contained in one of the first GUI windows 451-453, by a user. The user, typically a physician, but possibly also the patient, can then answer the questions displayed in the questionnaire dialog windows 454-456 by selecting/deselecting GUI elements such as checkboxes and radio buttons contained in the displayed electronic questionnaires. After having completed editing, selecting and/or deselecting GUI elements of the questionnaire dialog windows, a final score value is calculated in step 257 based on the additional data provided by the user via the questionnaire dialog windows. In decision 258, the final scover value is compared with a second threshold. In case it was determined that the second threshold was exceeded by the final SV, a positive second diagnosis result is displayed 259 in a second GUI window 457 depicted in fig. 4c.

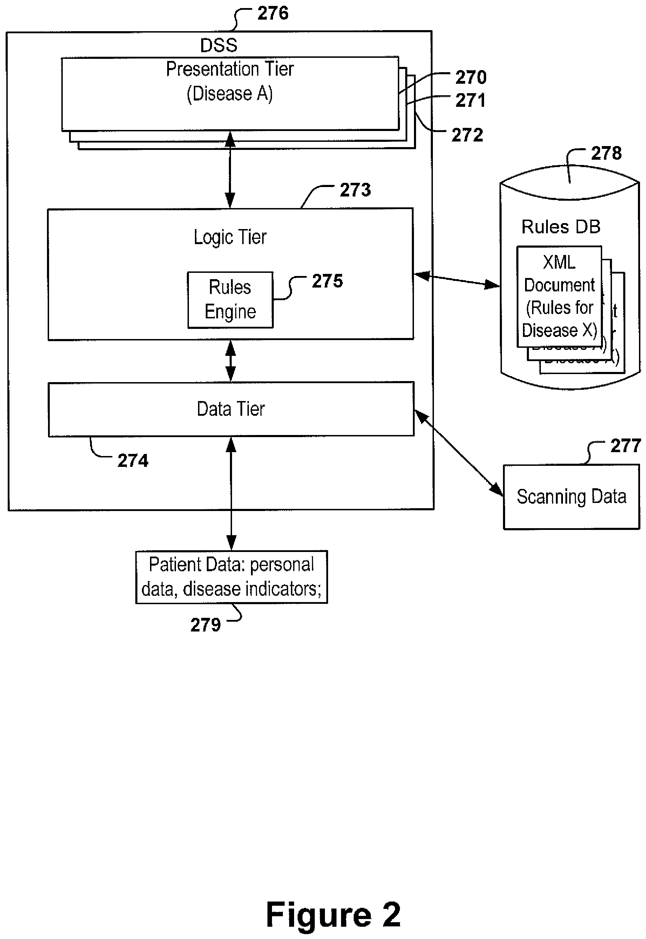

**Figure 2** depicts the architecture of the decision support system 276 according to embodiments of the invention. The decision support system comprises multiple tiers: a data tier 274 for accessing a database 279 comprising patient data, in particular personal data and disease indicators of the patient. The database 279 can be one or more relational databases or any other form of structured data repository. According to some embodiments, the data tier comprises an interface to a doctor information system 287. According to some embodiments, the data tier does not access the patient's database 279 directly but rather receives biomedical and other data of the patient via the doctor information system. The main purpose of the data tier 274 is to provide the logic tier 273 with biomedical data of one or more patients.

According to embodiments, the method depicted in figure 1 a is executed by the logic tier 273 which comprises a rule engine 275. The rule engine is able to access the rules repository 278 and to read all rules contained therein or to read selectively the rules for a particular disease in order to determine, whether said disease is present in a patient or not. According to some embodiments, the rules are stored in the rules repository in the form of XML documents. The received rules are evaluated on patient data (that is patient specific disease indicators) having been read by the data tier from database 279. According to some embodiments wherein the data tier provides an interface to a doctor information system, the logic tier executes the steps of the method whenever the user of the doctor information system opens a patient record or stores a new symptom or diagnosis to said record.

According to preferred embodiments, the logic tier does not comprise any disease-specific program logic.

It is solely the rules belonging to the third rule sets and the program logic of the presentation tier(s) which are disease specific and may require an update or replacement in case the available biomedical knowledge on the respective disease has considerably increased or changed. This feature is advantageous, because whenever new biomedical knowledge necessitating an adaptation of the disease prediction algorithm, i.e., the rules, is available, it is not necessary to rewrite or recompile the source code of the logic tier or the data tier. Only the presentation tier 270-272 may have to be updated in some cases, as its content specifies disease- and organ system specific questionnaire dialog widows, the second threshold and algorithms for final SV calculation.

Updating the rules of the first and second rule sets can easily accomplished without any code recompilation by simply loading new rule versions into the rules repository 278.

The presentation tier is used to present the first and second diagnosis results calculated by the decision support system to the user. According to preferred embodiments, the decision support system may comprise one or more presentation tiers 270, 271, 272, whereby for each particular disease on presentation tier is used. Each presentation tier is responsible for receiving the first diagnosis result from the logic tier and for presenting it to the user. Depending on the embodiment, this step may comprise receiving the TSV from the logic tier and displaying the first diagnosis result in dependence on a comparison of the TSV and a first threshold value. The diagnosis result may in addition comprise identifiers of those first rule sets havig positively contributed to the TSV. In addition, each presentation tier may be responsible for displaying, in dependence on the received diagnosis result, in particular on said first rule set identifiers, one or more questionnaire dialog windows. In addition, each presentation tier may be responsible for displaying windows showing a positive first diagnosis result and windows showing positive or negative second diagnosis results. Said windows may comprise additional GUI elements linking to additional information sources for the respective disease said presentation tier corresponds to.

The database 277 comprises scanning data, which is data being indicative of whether the rules for a particular disease, e.g. Morbus Fabry, have already been executed on the biomedical data of a particular patient or not. Before the decision support system evaluates the rules for a particular disease, it checks whether scanning data exists indicating that a scan has already been applied for said disease and said patient in the past. If this is the case, the evaluation of the rules is suppressed to save time and processing resources. The scanning data may be deleted when a new symptom or diagnose is added to the medical record of the patient. Database 277 can be implemented e.g. as SQLite database.

**Figure 3a** shows a client computer system 281 which comprises a working memory 291 and a processor 290 for executing computer readable instructions stored in computer readable storage medium 284. The instructions specify a multitier decision support system for determining, whether a disease, e.g. Morbus Fabry, is present in a patient or not as described above. The instructions may in addition specify a doctor information system 287 being interoperable with said decision support system. The client computer system 281 further comprises a graphical user interface 292 such as a computer screen. The client computer system 281 is operatively coupled to a database 279 comprising patients data, the rules repository 278 and a database comprising scanning data 277. The expression "operatively coupled" implies that said data resources may be integral part of the computer system 281, e.g. may be stored on storage medium 284, or maybe stored on another computer system and being accessible by the client computer system 281 via a network such as the Internet or an intranet af e.g. a hospital. The first and/or the second diagnosis result are displayed to a user 280 via graphical user interface 292.

**Figure 3b** depicts an alternative computer system architecture comprising a server computer system 282 and a client computer system 283 being connected to each other via network 288. The computer system 282 comprises a working memory 294 and a processor 293 for executing computer implemented instructions stored on storage medium 285. Said instructions encode a decision support system 276 which may consist of multiple tiers. The computer system 282 and its decision support system are operatively coupled to a database 277 comprising scanning data, to the rules repository 278 and to a database comprising biomedical patient data.

The client computer system 283 comprises a graphical user interface 297 for interaction with a user 280. It comprises working memory 296 and a processor 295 for executing computer implemented instructions stored to storage medium 286. Said computer implemented instructions may encode a doctor information system 287. The doctor information system is interoperable with the remote decision support system 276. In addition or alternatively, the doctor information system may be able to store patient related data, which may be gathered by a physician 280 from a patient, into database 279.

According to some embodiments, the doctor information system comprises or may be interoperable with a presentation tier 270 having been installed on the client computer system 283. The doctor information system receives a first diagnosis result calculated by the decision support system 276 on the server computer system 282 via network 288. The presentation of disease specific questionnaire dialog windows, the calculation of a final SV, the comparison of said final SV with a second threshold value and the display of a second diagnosis result in dependence on said comparison are in the responsibility of the presentation tier 270. This is advantageous, because the program code being particular to a particular disease such as Morbus Fabry is contained within one particular presentation tier. In case the biomedical knowledge on that disease grows and the calculation of the final SV and/or the contents of the questionnaires need to be updated, it is not necessary to change a single line of code in the data or logic tier of the DSS. Merely an update or replacement of the disease specific presentation tier on the server 282 or the client 283 may be required.

A multitude of additional client systems (not shown) may also store patient related data in a remote and centralized database 279. For example, the user 280 of the client computer system 283 may be a cardiologist while other client computer systems (not depicted in figure 3b) may be computer systems of physicians or hospitals specializing in the treatment of pain, the treatment of renal diseases or of other organ systems. According to embodiments, each of said client computer systems in addition comprises computer implemented instructions for generating a pseudonym from a user-selected secret and for using said pseudonym as access key for storing biomedical data of a patient into the remote central database 279.

**Figure 4a** depicts a set of GUI windows which are displayed to the user in dependence on the result of decision step 254. In case the calculated TSV value does not exceed the first threshold, no message is displayed to the user in order to avoid disturbing the user with a multitude of negative results. In case the calculated TSV value exceeds the first threshold, a first window 451-453 comprising a positive first diagnosis result is displayed to the user in step 255. According to embodiments, each first window comprises information on all first rule sets having positively contributed to the TSV. Depending on the embodiments, each first rule set may thereby be indicated by terms such as 'organ system' X, 'disease pattern' X or the like. One or more links allowing the user to open additional windows comprising additional information on the respective disease may also be contained in said first window.

According to preferred embodiments, said additional information is a short description of the disease, its causes and treatment options, which can be read within 100 seconds.

According to preferred embodiments, each first window 451-453 comprises one link to one or more further questionnaire dialog windows for collecting data to sharpen the diagnosis preciseness. Said questionnair dialog windows are preferentially encoded in the source code of the presentation tier corresponding to the disease whose presence in a patient is to be determined. Accordingly, the first diagnosis result displayed in any of the windows 451-453 depends on the total score value and on the type of first rule sets positively contributing to the TSV. In case multiple first rule sets relating e.g. to multiple different organ system returned a score value larger than zero, multiple questionnair dialog windows 454-456 may be displayed simultaneously or in a sequential order. Accordingly, which alternative is actually displayed depends on the first diagnosis result.

According to some embodiments, each first window 451-453 in addition comprises a 'pattern' related reference list allowing a user to retrieve additional information on the disease whose presen.

According to other embodiments, all questionnair dialog windows 454-456 shown in dependence on the first diagnosis result can bei eimplemented as sections within one single large questionnair dialog window. Said single large questionnair dialog window comprises, for each first rule set having positively contributed to the to the TSV, a separate window pane with one or more questions relating to the disease indicators of the respective first rule set. Each of said questions shown in association with a selectable GUI element, e.g. checkboxes or drop-down lists. According to some embodiments, the first rule set related questions in each window pane can be displayed or hidden in dependence on a user action, e.g. a click on a title of each window pane. In other words, the first rule set related questions are displayed as dynamically expandable tree structure. According to some of said embodiments, the one single large questionnair dialog window may in addition comprise questions and associated selectable GUI elements which are shown independently of the type of positively contributing first rule sets. Said questions may relate to questions regarding the family history of a patient.

According to embodiments, the logic tier of the DSS receives a disease identifier of the disease whose presence is to be determined in a patient. The logic tier selects a presentation tier corresponding to said disease in dependence on the received disease identifier and calculates a first diagnosis result by evaluating disease-related rules. According to embodiments, the first diagnosis result is assigned with the TSV, with identifiers for the first rule sets having positively contributed to the TSV, and with an identifier of the disease. The first diagnosis result is then forwarded to the selected presentation tier for displaying the first diagnosis result, for displaying questionnair dialog windows in dependence on the first rule set identifiers contained in said first diagnosis result and for calculating and displaying asecond diagnosis result by evaluating one or more third rule sets on the additional data gathered via said questionnair dialog windows. According to some embodiments, the disease specific presentation tier retrieves, for each received first rule set identifier, a third rule set from the rules repository and applies said rules on the additional data to calculate the final SV.

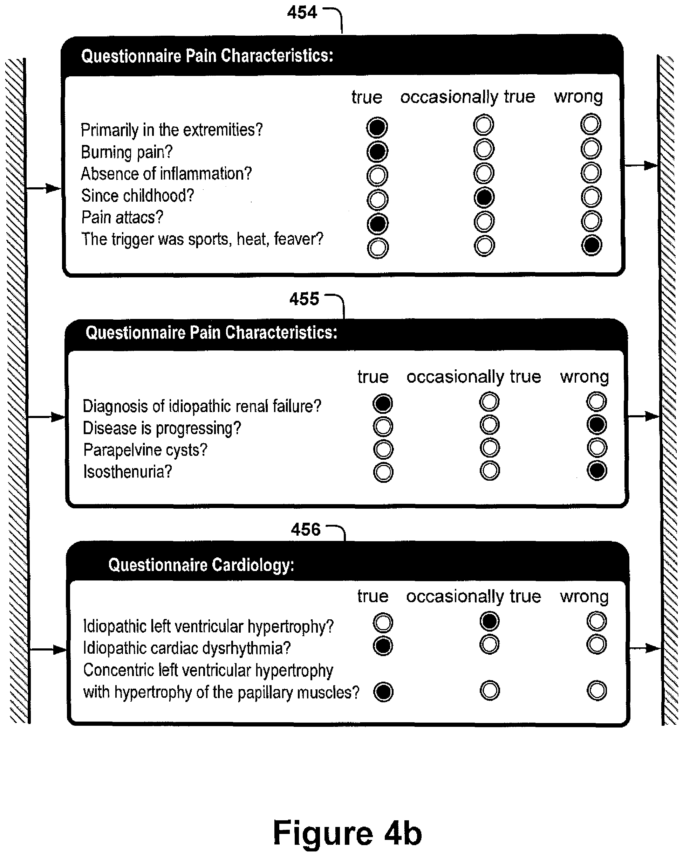

Windows 454, 455, and 456 as displayed in figure 4b show such organ specific questions The questionnaire dialogue windows 454-456 of figure 4b are mere examples. For each organ system, one or more questionnaire dialogue windows or separate window panes may be presented to a user in order to gather data for calculating the final score value.

According to some embodiments, each positively contributing first rule set corresponds to one or more organ specific dialog windows while according to other embodiments there exist at least some first rule set which do not trigger the display of a corresponding questionnair dialog window or trigger the display of one or more dialog windows comprising questions not related to a particular organ system.

In case the final SV exceeds the second threshold, a second window 457, is displayed to the user. Said window comprises a positive second diagnosis result comprising the information that the patient is predicted to have said disease.

Said window may also comprise additional information regarding the disease, recommended medication and/or treatment or billing information. In case it is determined in decision step 258 that the final SV does not exceed the second threshold, a negative second diagnosis result is displayed in third window 450 comprising the information that the presence of the examined disease, e.g. Morbus Fabry, is unlikely.

According to embodiments, all rules of all first rule sets of a particular disease, e.g. Morbus Fabry, are evaluated on biomedical patient data. In case the total score value obtained thereupon exceeds a first threshold of e.g. 79%, the corresponding positive result window is displayed. According to the score values of different rules listed in table is 1-8, a positive result window is displayed, for example, if

(a) the risk calculated for one first rule set/one organ system, e.g. the pain codes, is 80%, or
(b) the risk calculated for two rule sets/two organ systems, e.g. the pain codes and the renal system, are 40% respectively, or
(c) the risk calculated for three or more rule sets/three or more organ systems exceeds the first threshold.

GUI window 451 corresponds to option a), window 452 corresponds to option b), and window 453 corresponds to option c).

Depending on the embodiment, the decision support system can be implemented by means of any programming language such as, for example, Java or C#. A disease specific class, e.g. a Morbus Fabry class, may receive a copy of all disease specific first rule sets and a corresponding first threshold for displaying a positive result. An init-function of said class may trigger the display of a corresponding result window.

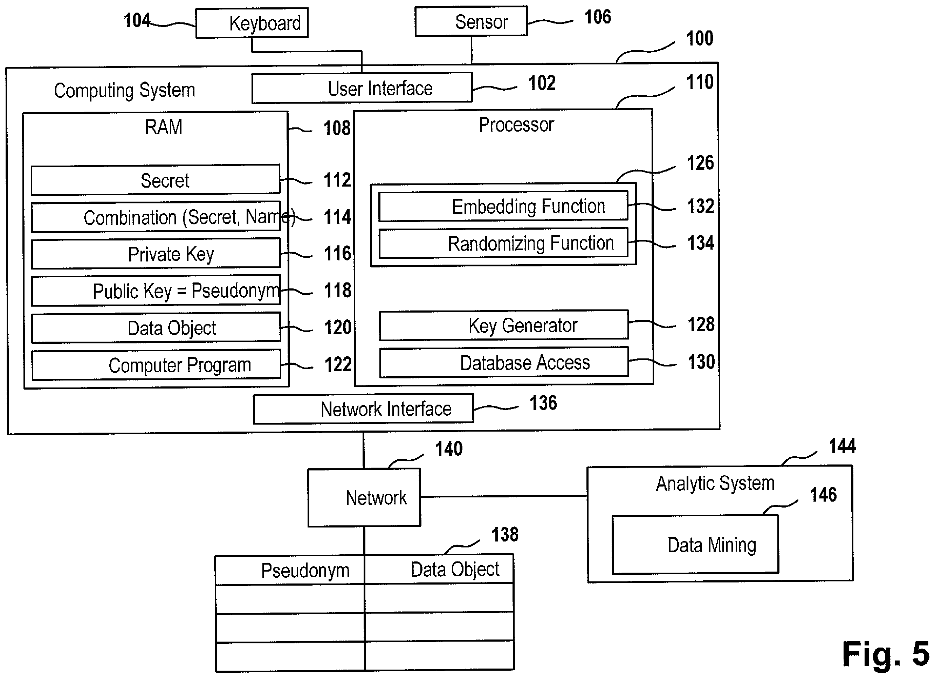

**Figures 5-13** will in the following describe how a pseudonym can be generated from a user-selected secret. That pseudonym can be used as database access key for storing biomedical data of a patient to a remote centralized database. This is advantageous, because a multitude of completely separated, independent clients, e.g. the computer systems of a multitude of different physicians specializing in different medical sub-disciplines, are able to store biomedical data of a patient in a secure and centralized form as one database record via one unique database access key without having to exchange any patient related data.

**Fig. 5** shows a computer system 100 that has a user interface 102 for a user's entry of a user-selected secret that is designated as $s_T$ in the following. For example, a keyboard 104 may be coupled to the computer system 100 for entry of $s_T$. Instead of a keyboard 104 a touch panel or another input device can be coupled to the computer system 100 for entry of $s_T$. In addition, a sensor 106 can be coupled to the computer system 100 such as for capturing biometric data from a biometric feature of the user. For example, the sensor 106 may be implemented as a fingerprint sensor in order to provide biometric fingerprint data to the computer system 100.

A public parameter, such as the user's name or email address, can also be entered into the computer system 100 via the keyboard 104 or otherwise. For example, a personal set $V_{T,i}$ containing at least one user-specific public parameter, such as the user's name or email address, is entered into the computer system 100 by the user $T_i$.

The computer system 100 has a memory 108, such as a random access memory, and at least one processor 110. The memory 108 serves for temporary storage of the user-selected secret $s_T$ 112, a combination 114 of $s_T$ 112 and $V_{T,i}$, a private key 116, a public key 118 that constitutes an access key for a database and/or pseudonym of the user $T_i$, and a data object 120, such as a medical data object containing medical data related to the user $T_i$. Further, the memory 108 serves for loading computer program instructions 122 for execution by the processor 110.

The computer program instructions 122 provide an embedding and randomizing function 126, a key generator 128 and may also provide a database access function 130 when executed by the processor 110.

The embedding and randomizing function 126 may be provided as a single program module or it may be implemented by a separate embedding function 132 and a separate randomizing function 134. For example, the embedding function 132 or an embedding component of the embedding and randomization function 126 provides the combination 114 by concatenating $s_T$ and the user's name or by performing a bitwise XOR operation on $s_T$ and the user's name.

In one implementation, the embedding and randomizing function 126 implements symmetric encryption provided by a symmetric cryptographic algorithm, e.g. AES, using a user-specific symmetric key for encryption of the user-selected secret 112. This provides both embedding and randomizing of $s_T$ 112.

In another implementation, the embedding function 132 is implemented by a binary cantor pairing function for embedding $s_T$ 112 and $V_{T,j}$, and the randomizing function 134 is implemented by AES encryption using a symmetric key that is the same for the entire set of users **T.**

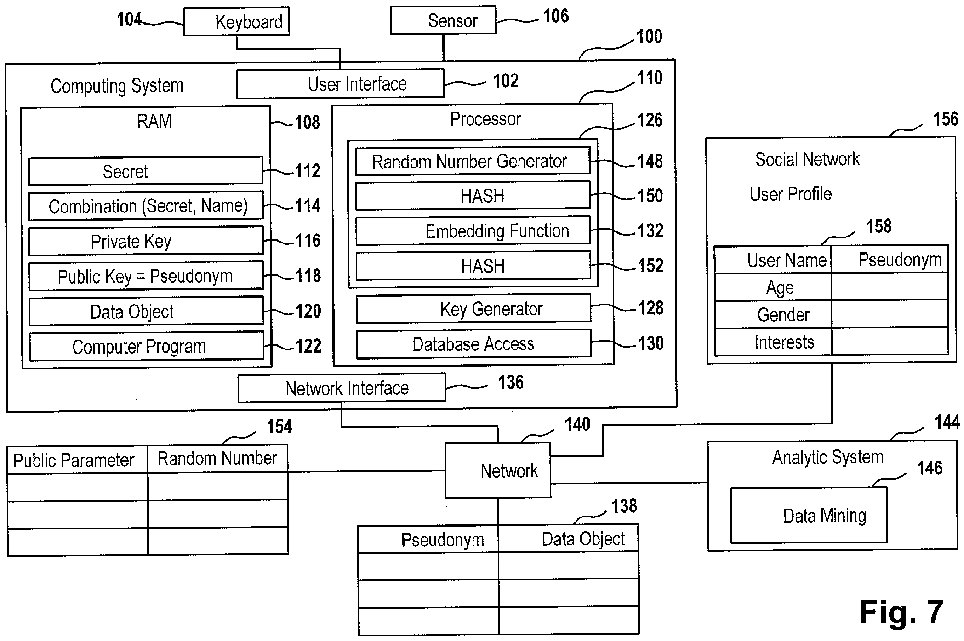

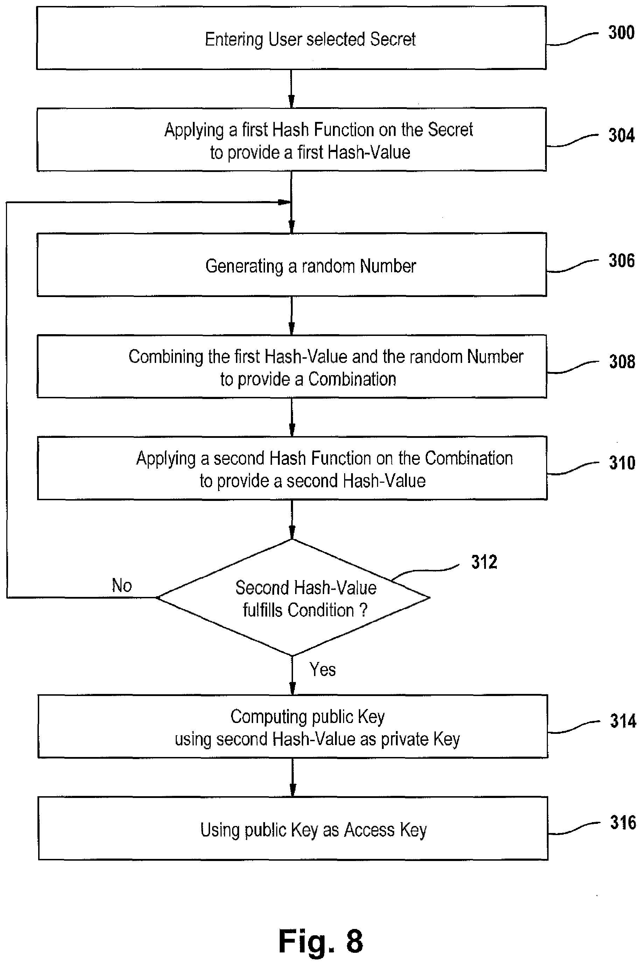

In still another embodiment the embedding and randomizing function 126 is implemented by two different hash functions and a random number generator (cf. the embodiment of Figs. 7 and 8).

The key generator 128 serves to compute public key 118 using elliptic curve cryptography (ECC). The base point given by the domain parameters of the elliptic curve is multiplied by the private key 116 which provides the public key 118. By varying the base point and leaving the other domain parameters of the elliptic curve unchanged multiple access keys and/or pseudonyms can be computed for the user $T_i$ on the basis of the same secret $s_T$.

The computer system 100 may have a network interface 136 for coupling the computer system 100 to a database 138 via a communication network 140, such as the Internet. The database access function 130 enables to perform a write and a read access for accessing the data object 120 stored in the database 138 using the public key 118, i.e. the user's pseudonym, as a database access key, e.g. a primary key, candidate or foreign key value that uniquely identifies tuples in a database relation.

Further, an analytic system 144, such as a decision support system (DSS) can be coupled to the database 138 such as via the network 140. The analytic system 144 comprises a component 146 for analyzing the data objects of the users T which are stored in the database 138, such as by data mining or data clustering.

In one application the data objects stored in the database 138 contain medical data of the various users. By analyzing the various data objects using techniques such as data mining and/or data clustering techniques medical knowledge can be obtained. For example, data clustering may reveal that certain user attributes contained in the medical data increase the risk for certain diseases.

For generating a pseudonym $p_{T,i}$ for a user $T_i$ based on the secret $s_T$ 112 and domain parameters $D_i$ containing a base point for the elliptic curve cryptography the following steps are executed by the computer system 100 in operation:

The user $T_i$ enters his or her user-selected secret $s_T$ 112 such as via the keyboard 104. In addition, the user may enter at least one public parameter $V_{T,i}$ such as his name or email address via the keyboard 104 or otherwise. Such a public parameter $V_{T,i}$ may also be permanently stored in the computer system 100.

The secret $s_T$ 112 is temporarily stored in the memory 108. Upon entry of the secret $s_T$ 112 the embedding function 132 or the embedding component of the embedding and randomizing function 126 generates the combination 114 of the secret $s_T$ 112 and the public parameter $V_{T,i}$. The resultant combination 114 is temporarily stored in the memory 108.

Next, the randomizing function 134 or the randomizing component of the embedding and randomizing function 126 is invoked in order to calculate the private key 116 on the basis of the combination 114. The resultant private key 116 is temporarily stored in memory 108. In the next step, the key generator 128 is started for computing the public key 118 by multiplying the base point contained in the domain parameters $D_i$ of the elliptic curve being used by the private key 116.

The public key 118, i.e. the pseudonym $p_{T,i}$, is stored in memory 108. The secret $s_T$ 112, the combination 114 as well as the private key 116 as well as any intermediate result obtained by execution of the embedding and randomizing function 126 and the key generator 128 are then erased from the memory 108 and/or the processor 110. As a consequence, there is no technical means to reconstruct the assignment of the resultant pseudonym to the user $T_i$ as only the user knows the secret $s_T$ 112 that has led to the generation of his or her pseudonym $p_{T,i}$. A data object 120 containing sensitive data of the user $T_i$, such as medical data, can then be stored by execution of the database access function 130 in the pseudonym database 138 using the pseudonym $p_{T,i}$ 118 as a database access key, e.g. a primary key or candidate key value that uniquely identifies tuples in a database relation.

The user-selected secret $s_T$ 112 may be obtained by combining a user-selected password or secret key with biometric data of the user $T_i$ that is captured by the sensor 106. For example, a hash value of the user-selected password or secret key is calculated by execution of respective program instructions by the processor 110. In this instance the hash value provides the user-selected secret $s_T$ 112 on which the following calculations are based.

A plurality of users from the public set of enrolled participants **T** may use the computer system 100 to generate respective pseudonyms $p_{T,i}$ and to store data objects containing sensitive data, such as medical information in the database 138 as it has been described above in detail for one of the users $T_i$ by way of example.

For reading the data object of one of the users $T_i$ from the database 138 the user has to enter the secret $s_T$ 112. Alternatively, the user has to enter the user-selected password or secret key via the keyboard 104 and an acquisition of the biometric data is performed using the sensor for computation of a hash value that constitutes $s_T$ 112. As a further alternative, the secret key is read by the computer system from an integrated circuit chip card of the user. On the basis of $s_T$ 112 the pseudonym can be computed by the computer system 100.

The pseudonym is then used for performing a database read access on the database 138 in order to read one or more data objects 120 that are stored in the database 138 for that user $T_i$. After the database access operation has been performed the secret $s_T$ 112, the combination 114, the private key 116 and the public key 118 are erased from the computer system 100 as well as any intermediate computational results.

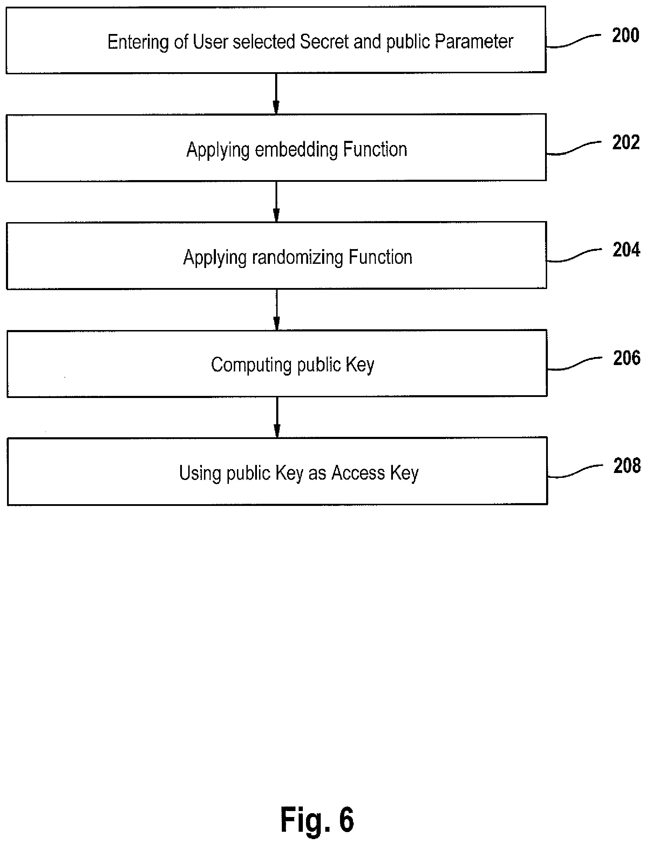

**Fig. 6** shows a corresponding flowchart.

In step 200 the user $T_i$ enters his or her user-selected secret $s_T$ and public parameter $V_{T,i}$. In step 202 $s_T$ and $V_{T,i}$ are combined to provide the first combination by the embedding function (cf. embedding function 132 of Fig. 5). Next, the randomizing function (cf. randomizing function 134 of Fig. 5). is applied on $s_T$ and $V_{T,i}$ in step 204 which provides a private key. As an alternative, an embedding and randomizing function 126 is applied on $s_T$ and $V_{T,i}$ which provides the private key.

In step 206 a public key is computed using the private key obtained in step 204 and the public key is used in step 208 as a pseudonym of the user $T_i$. For example the pseudonym may be used as a database access key, e.g. a primary key or candidate key value that uniquely identifies tuples in a database relation for storing a data object for the user $T_i$ in a database with pseudonymous data (cf. database 138 of Fig. 5).

**Fig. 7** shows a further embodiment of computer system 100. In the embodiment considered here the embedding and randomizing function 126 comprises an embedding function 132, a random number generator 148, a first hash function 150 and a second hash function 152. In the embodiment considered here the computation of the private key 116 based on $s_T$ 112 may be performed as follows:

The first hash function 150 is applied on the user-selected secret $s_T$ 112. This provides a first hash value. Next, a random number is provided by the random number generator 148. The random number and the first hash value are combined by the embedding function 132 to provide the combination, i.e. the embedded secret $s_T$ 112.

The combination of the first hash value and the random number can be obtained by concatenating the first hash value and the random number or by performing a bitwise XOR operation on the first hash value and the random number by the embedding function 132. The result is a combination on which the second hash function 152 is applied to provide a second hash value. The second hash value is the private key 116 on which the calculation of the public key 118 is based.

Dependent on the implementation it may be necessary to determine whether the second hash value fulfils one or more predefined conditions. Only if such conditions are fulfilled by the second hash value it is possible to use the second hash value as the private key 116 for the following computations. If the second hash value does not fulfill one or more of the predefined conditions a new random number is provided by the random number generator 148 on the basis of which a new second hash value is computed which is again checked against the one or more predefined conditions (cf. the embodiment of Fig. 8).

The random number on the basis of which the private key 116 and thereafter the public key 118 has been computed is stored in a database 154 that is coupled to the computer system 100 via the network 140. The random number may be stored in the database 154 using the public parameter $V_{T,i}$ as the database access key for retrieving the random number for reconstructing the pseudonym at a later point of time.

The user $T_i$ may use the pseudonym provided by the computer system 100 for his or her registration in an anonymous online community 156 e.g. a social network. For registration the user $T_i$ creates his or her user profile 158 by entering the pseudonym 118 as the username such that the various private data entered into the user profile 158 remain private even though they are published in the online community 156 due to the fact that the assignment of the pseudonym to the user $T_i$ is stored nowhere and cannot be reconstructed by technical means without knowledge of the user-selected secret $s_T$ 112.

For reconstructing the pseudonym the user has to enter his or her user-selected secret $s_T$ 112 into the computer system on the basis of which the first hash value is generated by the hash function 150 and the combination 114 is generated by the embedding function 132 or the embedding component of the embedding and randomizing function 126 using the first hash value and the random number retrieved from the database 154 by the use of the public parameter $V_{T,i}$.

Depending on the implementation, the user may also need to enter the user's public parameter $V_{T,i}$. A database access is performed using the user's public parameter $V_{T,i}$ as a database access key, e.g. a primary key or candidate key value that uniquely identifies tuples in a database relation, in order to retrieve the random number stored in the database 154.

In other words, the reconstruction of the private key 116 is performed by applying the embedding function 132 on the first hash value obtained from the user-selected secret $s_T$ 112 and the retrieved random number which yields the combination 114. The first hash value is combined with the random number retrieved from the database 154 by the embedding function 132 to provide the combination onto which the second hash function 152 is applied which returns the private key 116, out of which the public key 118, i.e. the pseudonym, can be computed. After the user $T_i$ has recovered his or her pseudonym a database access for reading and/or writing from or to the database 138 may be performed or the user may log into the online community 156 using his or her pseudonym for anonymous participation in the online community 156.

**Fig. 8** shows a respective flowchart for generating a pseudonym $p_{T,i}$ for user $T_i$. In step 300 the user enters the user-selected secret $s_T$. In step 304 a first hash function is applied on the user-selected secret $s_T$ which provides a first hash value. In step 306 a random number is generated and in step 308 an embedding function is applied on the first hash value and the random number to provide a combination of the first hash value and the random number. In other words, the first hash value and the random number are mapped to a 1-dimensional space, e.g. a single number, by the embedding function. The combination can be obtained by concatenating the random number and the first hash value or by performing a bitwise XOR operation on the first hash value and the random number.

In step 310 a second hash function is applied on the combination which provides a second hash value. The second hash value is a candidate for the private key. Depending on the implementation the second hash value may only be usable as a private key if it fulfils one or more predefined conditions. For example, if ECC is used, it is checked whether the second hash value is within the interval between 2 and n-1, where n is the order of the elliptic curve.

Fulfillment of such predefined conditions is checked in step 312. If the condition is not fulfilled, the control returns to step 306. If the condition is fulfilled, then the second hash value qualifies to be used as a private key in step 314 to compute a respective public key providing an asymmetric cryptographic key-pair consisting of the private key and the public key. In step 316 the public key computed in step 314 is used as a pseudonym such as for accessing a pseudomized database, participation in an anonymous online community or other purposes.

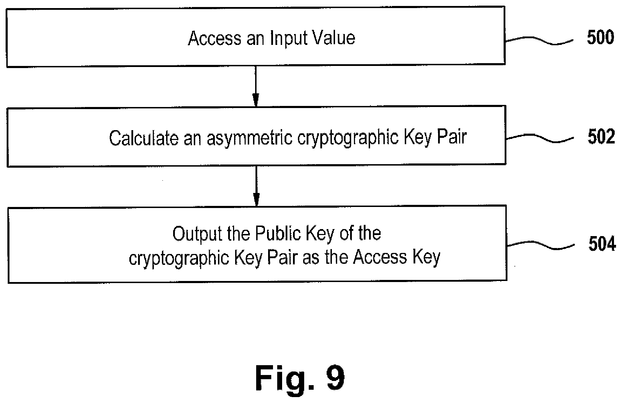

**Fig. 9** shows a block diagram which illustrates an embodiment of the method according to the invention. In step 500 an input value is accessed. The input value may be stored in a computer memory or computer storage device or the input value may be generated For example, the input value could be generated from a user-selected secret. In step 502 an asymmetric cryptographic key pair is calculated. The input value could be used to generate both the public and private keys, or the input value could also possibly be the private key. In step 504 the public key of the cryptographic key pair is outputted as the access key.

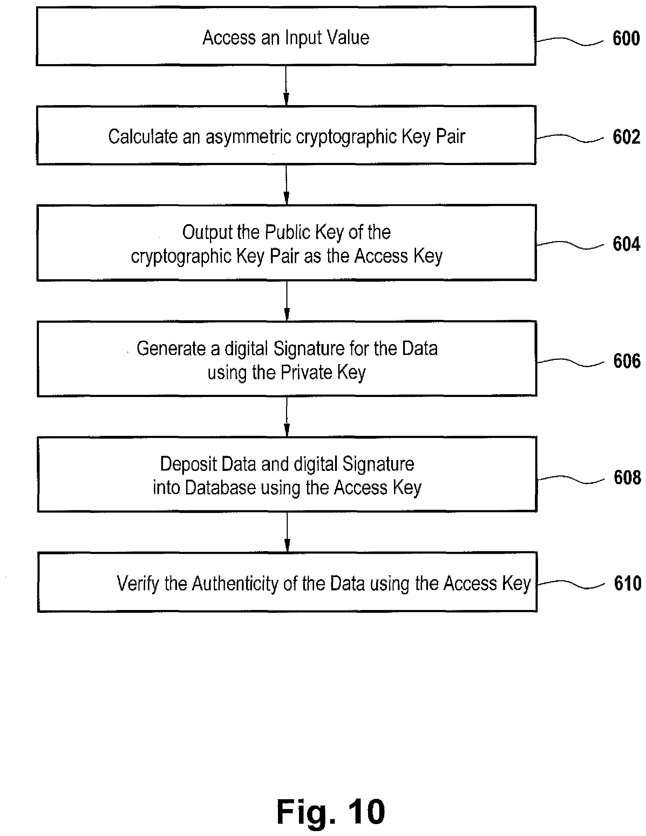

**Fig. 10** shows a further embodiment of the method according to the invention as a block diagram. In step 600 an input value is accessed. In step 602 an asymmetric cryptographic key pair is calculated. In step 604 the public key of the cryptographic key pair is outputted as the access key. In step 606 a digital signature for data which is to be deposited into a database is generated using the private key of the cryptographic key pair. In step 608 data is deposited along with the digital signature into a database using the access key. The access key may be used to grant access to the database or as a permission to write data into the database or it may also serve as a reference for the data being deposited into the database. In step 610 the authenticity of the data is verified using the access key. The access key is the complimentary public key to the private key. The private key was used to generate the digital signature for the data and the public key can be used to verify the digital signature.

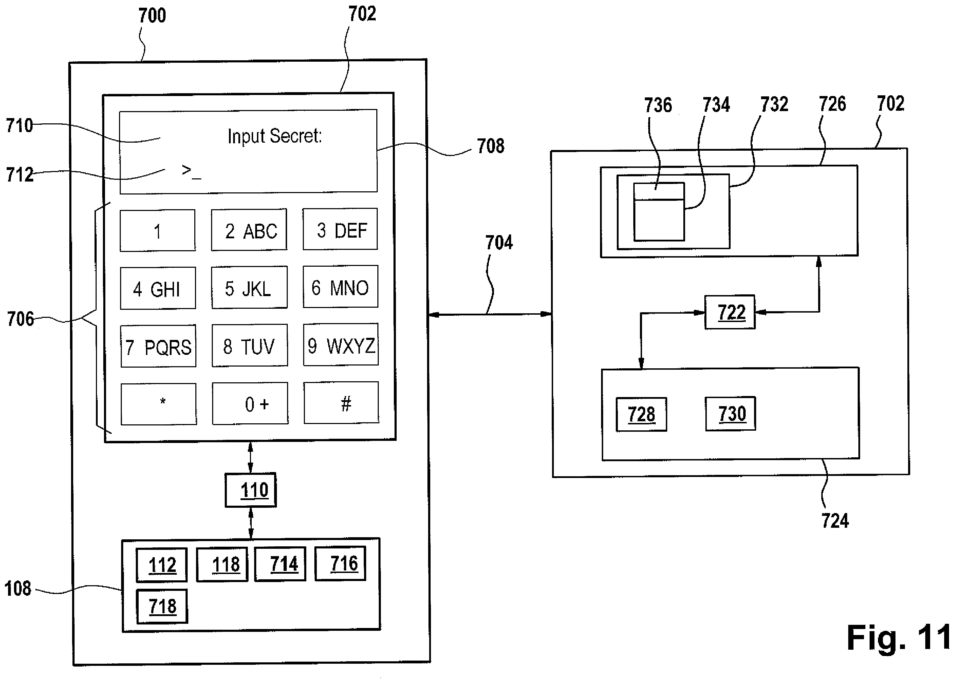

**Fig. 11** shows a functional diagram of a cell phone 700 according to an embodiment of the invention. The cell phone 700 is shown as being connected to a computer 702 via a communication link 704. The cell phone 700 may transfer a pseudonym 118 to the computer 702 via the communication link 704. In this embodiment the cell phone 700 could also be other types of mobile computing devices. These include for example, but are not limited to: a personal digital assistant, an mp3 player, and a laptop. The communications link 704 may be a variety of different types of communication link. It may be a wireless cell phone connection, it may be a Bluetooth connection, or it may be a wireless land connection, or it may be a LAN connection.

The cell phone 700 is shown as comprising a processor 110. The processor 110 is connected to a user interface 102 and a memory 108. The user interface 102 in this embodiment is shown as comprising a set of input keys 706 and a display 708. However, it is understood that the input 706 and the display 708 may be combined into a single functional unit. For instance many cellular telephones, personal digital assistants, and mp3 players use touch sensitive screens. Instead of using input keys 706 gestures or symbols on a touch sensitive screen may also be used. The display 708 shows a message 710 prompting a user to input a user-selected secret 112. The display 708 also shows a cursor 712 which shows a user where the value is input. The processor 110 is also connected to a memory 108. Within the memory is shown the stored user-selected secret 112. The user-selected secret 112 may be used to generate the input value 714. In some embodiments the user-selected secret 112 may be identical with the input value 714. In other embodiments the user-selected secret 112 may be used to generate the input value 714. An input value generator 716 may be used to generate an input value 714 from a user-selected secret 112. The input value 714 may be equivalent to the private key 716 as was discussed in the embodiments of Figs. 5 and 7. The memory 108 may also contain a cryptographic module 718 which uses the input value 714 to generate a pseudonym 118.

The memory 108 shown in Figs. 5 and 7 may be equivalent to the memory 108 shown in Fig. 11. The data shown within the RAM or memory 108 shown in Figs. 5 and 7 may also be stored within the RAM or memory 108 of Fig. 11. The processor 110 shown in Fig. 11 may also be equivalent to the processors shown in Figs. 5 and 7. That is to say that the processor 110 and the memory 108 of Fig. 11 may also be used to implement the embodiments shown in Figs. 5 and 7. The memory 108 shown in Figs. 5, 7, and 9 are embodiments of a computer readable storage medium.

In Fig. 11 there is a connection 704 between the cell phone 700 and the computer 702. The cryptographic module 718 is able to generate an access key 118 using the input value 714. The cell phone 700 is able to transmit the access key 118 to the computer 702 via the communications link 704. The computer system 702 comprises a processor 722, computer memory 724, and computer storage 726. The computer memory 724 contains a database application program 728 and data 730. A database application program is any program or set of computer executable instructions which uses, accesses, and/or modifies a database.

The database application program 728 may use the access key 118 from the cell phone 700 to place data 730 into a database 732 which is contained within the computer storage 726. Shown within the database 732 is the data 734 after it has been placed into the database 732. In this case the access key 736 is stored with the data 734. The access key could either be appended to the data 734 or it could be referenced to the data 734. During use the cell phone 700 could be used to generate an access key 118 when a user wishes to store and/or modify data 730, 734 into the database 732. For instance a user could use his or her cell phone to produce an access key 118 which is used for permission to post data to a bulletin board system or a social networking site. In another instance the cell phone 700 could be used to provide verification for a financial transaction. The data 730 may represent a request for a financial transaction.

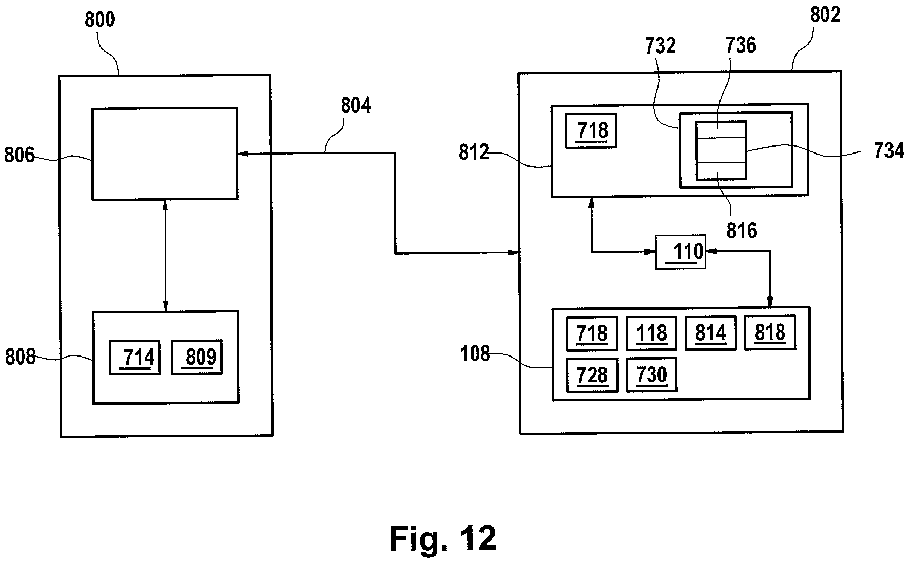

**Fig. 12** shows an embodiment of a computing device comprising a security token 800 and a computer 802. The security token 800 is connected to the computer 802 via a communications link 804. The communications link varies depending upon the implementation of the security token 800. For instance the security token may be an RFID tag in which case the communications link 804 is a radio frequency communications link. Alternatively, the security token 800 may also be something as simple as a USB thumb drive. In this case the communications link 804 is a USB bus. The security token 800 is shown as comprising a microcontroller 806 and a memory 808. Memory 808 is shown as containing the input value 714 and an access control module 809. The access control module 809 is optional, but the access control module 809 contains instructions for operation of the microcontroller 806 which control access to the input value 714.

The security token 800 may be constructed such that the input value 714 is stored in secure memory or memory which may be destroyed if the security token 800 is disassembled. The computer 802 comprises a processor 110 and computer memory 108. The computer 802 also comprises computer storage 812. During operation the processor 110 may access via the communications link 804 the input value 714 stored in memory 808. The processor 110 may then use a cryptographic module 718 to generate the access key 118. The access key 118 may be used as a pseudonym in some embodiments.

The cryptographic module 718 is also shown as being stored in the computer storage 812. Both the computer memory 108 and the computer storage 812 are examples of computer readable storage medium. The embodiments of Figs. 5 and 7 may be implemented using the security token 800 and computer 802 of Fig. 12. For instance the processors 110 of Figs. 5 and 7 may correspond to the processor 110 of Fig. 12. The memory 108 of Figs. 5 and 7 may also correspond to the memory 108 of Fig. 12. The data and instructions shown as being stored in the memory 108 or the processor 110 may also be stored in the processor 110 or memory 108 of Fig. 12 respectively.

In the computer of Fig. 2 the cryptographic module 718 is adapted for using the input value 714 for generating an access key 118. The access key is shown as being located within the computer memory 108. The cryptographic module 718 can use the input value 714 to generate a private key 818. The private key 818 can be used to calculate a digital signature 814. The access key 118 can be used by a database application program 728 to enter the data 730 into database 732 which is located within the computer storage 812. Within the database 732 the data 730 is represented by the numeral 734. The data 734 has had the access key 736 and the digital signature 816 either appended to or referenced to the data 734. In this embodiment the data 734 contains a digital signature 816 which could be used to verify the authenticity and/or authorship of the data using the access key 736 (which functions also as a public key). In this embodiment the security token 800 can be used for depositing data into a database 732 or other file in a way which merely identifies the origin and authenticity of the data 734 without revealing the author's identity.

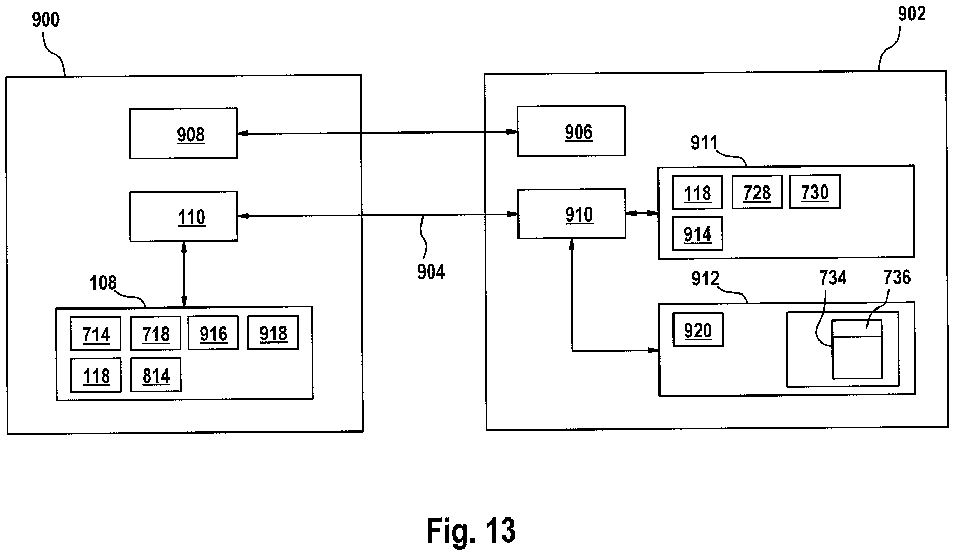

**Fig. 13** shows an embodiment of a smart card 900 according to an embodiment of the invention. The smart card 900 is shown as being connected to a computer 902 via a communications link 904. The nature of the communications link 904 depends upon how the smart card 900 is implemented. For instance if the smart card 900 connects to the computer 902 via contacts or electrical connections then the communications link 904 is simply a computer bus. However, if the smart card 900 uses an RFID communications link then the communications link 904 to the computer 902 is via radio.

The smart card 900 in this embodiment is shown as being powered by the computer 902. The computer 902 comprises an electrical power source 906 which is connected to an electrical power receiver 908. In the case of electrical contacts then this is simply an electrical connection between the two of them. For an RFID smart card the connection between the electrical power source 906 and the electrical power receiver 908 is through electrical induction. The electrical power receiver 908 powers the smart card 900. The smart card 900 is shown as comprising a processor 110. The processor 110 is connected to a computer memory 108. The computer memory 108 contains the input value 714 in a secure memory location. There is a cryptographic module 718 which may be used to generate the public key 118 or access key. The access key may be a pseudonym. The processor 110 is connected to a processor 910 of the computer system 902. The computer system 902 is shown as comprising computer memory 911 and computer storage 912.

During operation the processor 910 may request an access key 118 from the processor 110 of the smart card 900. The access key 118 may be a pseudonym. The computer system 902 may comprise a smart card access module 920 which comprises instructions or commands which allow basic access to the smart card 900. When access is granted the processor 110 will use the input value 714 and the cryptographic module 718 to calculate the pseudonym 118. In this embodiment, the pseudonym is generated using elliptical curve cryptography. The pseudonym is generated using a first base point 916.

The processor 910 may also request a public encryption key 914 to be generated by the smart card 900. In this case the processor 110 uses the second base point 918 which is stored with the memory 108 and the cryptographic module 718 to generate the public encryption key 914 which is output to the processor 910. In this example the pseudonym 118 and the public encryption key 914 are both shown as being stored in computer memory 911.

The smartcard 900 uses the input value 714 with the cryptographic module 718 to generate the access key 118. The access key 118 can be communicated with the computer 902 via the communications link 904. The database application program 728 is able to use the access key 118 to access a database 732 within the computer storage 912. In this figure it is shown that the data 730 has been stored in the database 732 and is referenced as data 734. In this embodiment the data 734 is referenced by the access key 736.

The embodiments of Figs. 5 and 7 may be implemented using the smart card 900 of Fig. 13. For instance the processors 110 of Figs. 5 and 7 may correspond to the processor 110 of Fig. 13. The memory 108 of Figs. 5 and 7 may also correspond to the memory 108 of Fig. 13. The data and instructions shown as being stored in the memory 108 or the processor 110 of Figs. 5 and/or 7 may also be stored in the processor 110 or memory 108 of Fig. 12.

**Mathematical appendix**

**1. Embedding functions.**

There exist n-ary scalar functions

$$d: \mathbb{N} \times ... \times \mathbb{N} \to \mathbb{N}$$

which are injective - and even bijective, where $\mathbb{N}$ is the set of natural numbers. The function d() embeds uniquely an n-dimensional space, i.e. n-tuples $(k_1,...,k_n)$, into scalars, i.e. natural numbers k.

**2. The binary cantor pairing function**

The binary cantor pairing function $\pi$ is an embodiment of embedding function 132. The binary cantor pairing function is defined as follows:

$$\pi: \mathbb{N} \times \mathbb{N} \to \mathbb{N}$$

$$\pi(m,n) = \frac{1}{2}(m+n)(m+n+1) + n$$

which assigns to each fraction $\dfrac{m}{n}$ the unique natural number $\pi(m, n)$ - thus demonstrating that there are no more fractions than integers. Hence, if we map both $s_T$ and $V_{T,i}$ to natural numbers and use the fact that all identities are distinct then $\pi(s_T, V_{T,i})$ yields a unique value for each identity, even if there are equal personal secrets. To be more precise, since this function does not distinguish between e.g. $\dfrac{1}{2}$, $\dfrac{2}{4}$ etc, it assigns to each fraction an infinite number of unique natural numbers.

**3. Elliptic curve cryptography (ECC)**

Let:

- p be a prime number, p > 3, and $\mathbb{F}_p$ the corresponding finite field
- a and b integers

Then the set E of points (x, y) such that

$$E = \{(x, y) \in \mathbb{F}_p \times \mathbb{F}_p \mid y^2 = x^3 + ax + b \} \qquad \text{(F1)}$$

defines an elliptic curve in $\mathbb{F}_p$. (For reasons of simplicity, we skip the details on E being non-singular and, as well, we do not consider the formulae of elliptic curves over finite fields with p = 2 and p = 3. The subsequent statements apply to these curves, too.)

The number m of points on E is its order.

Let P,Q $\in$ E be two points on E. Then the addition of points

$$P + Q = R \text{ and } R \in E \qquad (F2)$$

can be defined in such a way that E forms an Abelian group, viz, it satisfies the rules of ordinary addition of integers. By writing

$$P + P = [2]P$$

We define the k-times addition of P as [k]P, the point multiplication.

Now EC-DLP, the elliptic curve discretionary logarithm problem, states that if

$$Q = [k]P \qquad (F3)$$

then with suitably chosen a, b, p and P, which are known to the public, and the as well known to the public point Q it is computationally infeasible to determine the integer k.

The order n of a point P is the order of the subgroup generated by P, i.e. the number of elements in the set

$$\{P, [2]P, ..., [n]P\} \qquad (F4)$$

With all this in mind we define an elliptic curve cryptographic (ECC) system as follows. Let:

- E be an elliptic curve of order m
- B $\in$ E a point of E of order n, the base point

Then

$$D = \{a, b, p, B, n, co(B)\} \qquad (F5)$$

with $co(B) = \dfrac{m}{n}$ defines a set of domain ECC-parameters. Let now g be an integer and

$$Q = [g]B \qquad (F6)$$

Then (g, Q) is an ECC-key-pair with g being the private key and Q the public key.

For we rely on findings of Technical Guideline TR-03111, Version 1.11, issued by the Bundesamt fur Sicherheit in der Informationstechnik (BSI), one of the best accredited sources for cryptographically strong elliptic curves, we can take that m = n, i.e. co(B) = 1, and hence reduce (F5) to

$$D = \{a, b, p, B, n\} \qquad (F7)$$

Now we can define our one-way function. Let D be a set of domain parameters concordant with (F7). Then

$$f: [2, n-1] \rightarrow E$$

$$k \mapsto [k]B \tag{F8}$$

i.e. the point multiplication (F6), is an injective one-way function.

### 4. Implementing key generator based on ECC

The key generator 128 (cf. Figs. 5 and 7) can be implemented using ECC.

Definitions:

- There are public sets of ECC-domain parameters $D_1$, $D_2$,... concordant with (F7)

$$D_i = \{a_i, b_i, p_i, B_i, n_i\} \tag{F9}$$

- There are public functions: an embedding function d(), a randomising function r() and our one-way function f() defined by (F8).

- There is a public set of enrolled participants (users)

$$\mathbf{T} = \{T_1, T_2,...\} \tag{F10}$$

Note that a $\mathbf{T}_i$ does not necessarily possess any personally identifying details, i.e. we assume that $\mathbf{T}$ resembles the list of participants in an anonymous Internet-community, in which each participant can select his name at his discretion as long as it is unique.

- Each participant $T \in \mathbf{T}$ chooses at his complete discretion his personal secret $s_T$. In particular, for this secret is never revealed to anybody else - it is the participant's responsibility to ensure this - it is not subject to any mandatory conditions, such as uniqueness.

- Our pseudonym derivation function is

$$h() = f(r(d())) \tag{F11}$$

with the following properties:

- Given a $T \in \mathbf{T}$ with his $s_T$, a $D_i$ and $T, D_i \in V_{T,i}$

$$r(d(s_T, V_{T,i})) = g_{T,i} \tag{F12}$$

where $g_{T,i}$ is a unique and strong, i.e. sufficiently random, private ECC-key for $D_i$.

- The pseudonym $p_{T,i}$ corresponding to $T$, $s_T$ and $D_i$ is

$$p_{T,i} = f(g_{T,i}, D_i) = [g_{T,i}]B_i = (x_{T,i}, y_{T,i}) \tag{F13}$$

- There is a public set of pseudonyms

$$P = \{p_1, p_2, \ldots\} \qquad (F14)$$

such that **P** comprises one or more pseudonyms for each participant in **T** computed according to (F11). This wording implies that here is no recorded correspondence between a participant in **T** and his pseudonyms in **P,** i.e. each $p_{T,i}$ is inserted in an anonymous way as $p_k$ into **P.**

Remarks:

- The use of multiple domain parameters enables us to endow a single participant with a single personal secret with multiple pseudonyms. This in turn enables a participant to be a member of multiple pseudonymous groups such that data of these groups cannot - for, e.g. personal or legal reasons - be correlated. Therefore, attempts to exploit combined pseudonymous profiles for unintended, possibly malicious purposes, are of no avail.

- The distinction between two sets of domain parameters $D_i$ and $D_j$ can be minor. In accordance with our principle to use only accredited domain parameters, e.g. those listed in BSI TR-03111, we can set

$$D_i = \{a, b, p, B, n\} \qquad (F15)$$

by swapping B for a statistically independent $B_2$, i.e. by choosing a different base point, we can set

$$D_j = \{a, b, p, B_2, n\} \qquad (F16)$$

For $D_i$ and $D_j$ refer to the same elliptic curve we can have only one function (F12) and introduce the crucial distinction with (F13). This vastly simplifies concrete implementations - we select a suitable curve and vary the base points only.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfiill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Listof Reference Numerals

| | |
|---|---|
| 100 | Computer system |
| 102 | User interface |
| 104 | Keyboard |
| 106 | Sensor |
| 108 | Memory |
| 110 | Processor |
| 112 | A user-selected secret |
| 114 | Combination |
| 116 | Private Key |
| 118 | Public Key |
| 120 | Data Object |

| | |
|---|---|
| 122 | Computer program instructions |
| 126 | Embedding and randomizing function |
| 128 | Key Generator |
| 130 | Database access function |
| 132 | Embedding function |
| 134 | Randomizing function |
| 136 | Network interface |
| 138 | Database |
| 140 | Network |
| 144 | Analytic system |
| 146 | Component |
| 148 | Random number generator |
| 150 | Hash function |
| 152 | Hash function |
| 154 | Database |
| 156 | Online community |
| 158 | User profile |
| 200-208 | steps |
| 250-253 | steps |
| 254 | decision |
| 255-259 | steps |
| 270 | presentation coyote for first disease |
| 271 | presentation tier for second disease |
| 272 | presentation tier for said disease |
| 273 | logic tier |
| 274 | data tile |
| 276 | decision support system |
| 277 | scanning data |
| 278 | rules repository |
| 279 | patient data |
| 280 | user |
| 281 | client computer system |
| 282 | server computer system |
| 283 | client computer system |
| 284 | storage medium |
| 285 | storage medium |
| 286 | storage medium |
| 287 | doctor information system |
| 288 | network |
| 290 | processor |
| 291 | working memory |
| 292 | GUI |
| 293 | processor |
| 294 | working memory |
| 295 | processor |
| 296 | working memory |
| 297 | GUI |
| 300-310 | steps |
| 312 | decision |
| 314-316 | steps |
| 450 | third GUI window with negative second diagnosis result |
| 451 | first GUI window (positive first diagnosis result organ system I) |
| 452 | first GUI window (positive first diagnosis result organ system II) |
| 453 | first GUI window (positive first diagnosis result organ system II) |
| 454 | GUI window (questionnaire pain characteristics) |
| 455 | GUI window (questionnaire renal system) |
| 456 | GUI window (questionnaire cardiology) |
| 457 | third GUI window with positive second diagnosis result |

| | |
|---|---|
| 500-504 | steps |
| 600-610 | steps |
| 700 | cell phone |
| 702 | computer |
| 704 | communication link |
| 706 | input keys |
| 708 | display |
| 710 | message |
| 712 | cursor |
| 714 | input value |
| 716 | input value generator |
| 718 | cryptographic module |
| 722 | processor |
| 724 | computer memory |
| 726 | computer storage |
| 728 | database application program |
| 730 | data |
| 732 | database |
| 734 | data |
| 736 | access key |
| 800 | security token |
| 802 | computer |
| 804 | communications link |
| 806 | microcontroller |
| 808 | memory |
| 809 | access control module |
| 812 | computer storage |
| 814 | digital signature |
| 816 | digital signature |
| 818 | private key |
| 900 | smart card |
| 902 | computer |
| 904 | communications link |
| 906 | electrical power source |
| 908 | electrical power receiver |
| 910 | processor |
| 911 | computer memory |
| 912 | computer storage |
| 914 | public encryption key |
| 916 | first base point |
| 918 | second base point |
| 920 | smartcard access module |

**Claims**

1. A computer-implemented method for determining the presence of a disease in a patient, the method comprising:

- collecting biomedical data of a patient by executing the steps:

• receiving parts of the biomedical patient data of the patient from a plurality of different data sources (283), whereby each received part of the biomedical patient data comprises a unique identifier, the unique identifier being identical in each of the received parts of the biomedical patient data,
• storing the received biomedical data into one database (279), thereby using the unique identifier as database access key for all the received parts of the biomedical patient data,
whereby the unique identifier is generated by each of the data sources by executing the steps of:

- receiving (200) a user-selected secret (112);

33

- storing the user-selected secret in a memory (148);
- computing (202, 204) a private key (116) by applying an embedding (132) and randomizing function (134) onto the secret;
- storing the private key in the memory;
- computing (206, 502, 602) a public key (118) by using the private key, whereby the computation of the public key is performed by using a cryptographic one-way function, the public key and the private key forming a asymmetric cryptographic key pair;
- outputting (208, 316, 504, 604) the public key for providing the access key; and
- erasing the secret and the private key from the memory

- Receiving (250), by a computer system (282), first rule sets comprising rules, the rules of each first rule set being operable to evaluate a set of disease indicators, the disease indicators of each first rule set being biomedically related to each other, the rules of each first rule set being grouped into one or more second rule sets, each second rule set comprising a score value, said score value being indicative of the risk of the patient to have the disease in case the evaluation of the one or more rules of said second rule set on the biomedical data of the patient returns a positive result, wherein the totality of conditions contained in some rules of one of the second rule sets is a subset of the conditions contained in rules of another one of the second rule sets of a particular first rule set;
- Determining (251), by the computer system, for each first rule set, the highest score value of its second rule sets by evaluating all rules of said second rule sets on the biomedical data of the patient;
- Calculating (252), by the computer system, a total score value for the disease as a derivative of the determined highest score values of all first rule sets;
- Returning (253), by the computer system, a first diagnosis result, the first diagnosis result being indicative of the presence of the disease in the patient, the first diagnosis result having assigned the total score value.

2.  The computer-implemented method of claim 1,

    - whereby each first rule set represents one organ system and wherein each first rule set solely comprises rules for evaluating disease indicators to become manifest in said organ system, or
    - whereby each first rule set represents a set of biomedically related disease indicators and wherein each first rule set solely comprises rules for evaluating said disease indicators, or
    - whereby each first rule set represents one medical sub-discipline and wherein each first rule set solely comprises rules which comprises at least one condition on disease indicators of diseases examined in said medical sub-discipline.

3.  The computer-implemented method of claim 1 or 2, further comprising the step of displaying the first diagnosis result to a user, said displaying step comprising:

    - Determining (254), whether the total score value exceeds a first threshold value;
    - In case the total score value does exceed the first threshold value, executing the following steps:

        • Displaying (255) the first diagnosis result in a first GUI window (451, 452, 453);
        • Displaying (256) one or more dialog windows (454, 455, 456) prompting a user to enter additional data of the patient;
        • Calculating a final score value by evaluating one or more third rule sets on the entered additional data;
        • Displaying (259) positive positive second diagnosis result in a second GUI window (457) in case the final total score value exceeds a second threshold value, and
        • Displaying a negative second diagnosis result in case the final score value does not exceed the second threshold value.

4.  The computer-implemented method of claim 3, whereby the method is implemented in a decision support system (276) having a multi-tier architecture, whereby a first tier (274) provides for access to the biomedical patient data, whereby a second tier (273) comprises a rule engine and is operable to evaluate the rules of the first rule sets irrespective of the type of disease whose presence is to be determined, and whereby a third tier (270, 271, 272) displays the first and second diagnosis results to a user, whereby the number and type of dialog windows created by the third tier depends on an identifier of the disease and on the identifiers of all first rule sets having a highest score value larger than 0.

5. The computer-implemented method of claim anyone of claims 1-4, whereby the method for returning a diagnosis of a patient is implemented as a decision support system (276) on a computer system (281, 282), the decision support system being operatively coupled to a database (279) comprising the biomedical patient data of the patient, whereby the rules of each first rule set are evaluated on said biomedical data at an event being selected from the group consisting of:

- the moment of starting a doctor information system (287) being operatively coupled to the decision support system;
- the moment of accessing the biomedical patient data of the patient by the decision support system or by a doctor information system being operatively coupled to the decision support system; and
- at a predefined time and date.

6. The computer-implemented method of anyone of claims 1-5, whereby evaluating the one or more rules of each first rule set further comprises the steps of:

- after having evaluated the rules, storing information indicating that the rules have been evaluated;
- before evaluating the rules, checking whether said information has already been stored and evaluating the rules only in case said information has not yet been stored.

7. The computer-implemented method of anyone of claims 1-6, whereby the rules of the first rule sets are received by a decision support system from a rules repository (278), whereby the rules are stored and transmitted from the rules repository to the decision support system in a data format, said data format allowing to modify or exchange the rules without introducing changes to the decision support system or the structure of the rules repository.

8. The computer-implemented method of claim 3, whereby the rules of the first rule sets are received by a decision support system together with the first threshold.

9. A computer readable storage medium having stored therein instructions, which when executed by a computing device, cause the computing device to perform the steps of claim 1.

10. A computing system (278, 279, 281, 282, 283) comprising means for carrying out the method of claim 1.

## Patentansprüche

1. Computerimplementiertes Verfahren zum Bestimmen des Vorhandenseins einer Krankheit in einem Patienten, wobei das Verfahren Folgendes aufweist:

- Erfassen biomedizinischer Daten von einem Patienten durch Ausführen der folgenden Schritte:

• Empfangen von Teilen der biomedizinischen Patientendaten des Patienten aus mehreren verschiedenen Datenquellen (283), bei dem jeder empfangene Teil der biomedizinischen Patientendaten einen eindeutigen Identifikator aufweist, wobei der eindeutige Identifikator in jedem der empfangenen Teile der biomedizinischen Patientendaten identisch ist,
• Speichern der empfangenen biomedizinischen Daten in einer Datenbank (279), wodurch der eindeutige Identifikator als Datenbankzugangsschlüssel für alle die empfangenen Teile der biomedizinischen Patientendaten verwendet wird,
wobei der eindeutige Identifikator von jeder der Datenquellen durch Ausführen der folgenden Schritte erzeugt wird:

- Empfangen (200) eines benutzergewählten Geheimnisses (112);
- Speichern des benutzergewählten Geheimnisses in einem Speicher (108);
- Berechnen (202; 204) eines privaten Schlüssels (116) durch Anwenden einer Einbettungs- (132) und Randomisierungsfunktion (134) auf das Geheimnis;
- Speichern des privaten Schlüssels im Speicher;
- Berechnen (206, 502, 602) eines öffentlichen Schlüssels (118) durch Verwenden des privaten Schlüssels, bei dem das Berechnen des öffentlichen Schlüssels durch Verwenden einer kryptographischen Einwegfunktion durchgeführt wird, wobei der öffentliche Schlüssel und der private Schlüssel ein asymmetrisches kryptographisches Schlüsselpaar bilden;
- Ausgeben (208, 316, 504, 604) der öffentlichen Schlüssel zum Bereitstellen des Zugangsschlüssels und
- Löschen des geheimen und des privaten Schlüssels aus dem Speicher,

- durch ein Computersystem (282) Empfangen (250) erster Regeln aufweisender Regelsätze, wobei die Regeln jedes ersten Regelsatzes zum Auswerten eines Satzes von Krankheitsindikatoren funktionell sind, die Krankheitsindikatoren jedes ersten Regelsatzes biomedizinisch miteinander verwandt sind, die Regeln jedes ersten Regelsatzes in einen oder mehr zweite Regelsätze gruppiert sind, jeder zweite Regelsatz einen Bewertungswert aufweist, der genannte Bewertungswert das Risko des Patienten, die Krankheit zu haben, erkennen lässt, falls die Auswertung der einen oder der mehr Regeln des genannten zweiten Regelsatzes an den biomedizinischen Daten des Patienten ein positives Ergebnis erbringt, wobei die Gesamtheit von in einigen Regeln von einem der zweiten Regelsätze enthaltenen Bedingungen eine Teilmenge der in Regeln von einem weiteren der zweiten Regelsätze eines jeweiligen ersten Regelsatzes enthaltenen Bedingungen ist;
- durch das Computersystem Bestimmen (251) für jeden ersten Regelsatz des höchsten Bewertungswerts seiner zweiten Regelsätze durch Auswerten aller Regeln der genannten zweiten Regelsätze an den biomedizinischen Daten des Patienten;
- durch das Computersystem Ermitteln (252) eines Gesamtbewertungswerts für die Krankheit als eine Ableitung des bestimmten höchsten Bewertungswertes aller ersten Regelsätze;
- durch das Computersystem Erbringen (253) eines ersten Diagnoseergebnisses, wobei das erste Diagnoseergebnis das Vorhandensein der Krankheit in dem Patienten erkennen lässt, wobei dem ersten Diagnoseergebnis der Gesamtbewertungswert zugeordnet ist.

2. Computerimplementiertes Verfahren nach Anspruch 1,

- bei dem jeder erste Regelsatz ein Organsystem repräsentiert und wobei jeder erste Regelsatz ausschließlich Regeln zur Auswertung von Krankheitsindikatoren, die sich in dem genannten Organsystem manifestieren werden, aufweist oder
- bei dem jeder erste Regelsatz einen Satz biomedizinisch verwandter Krankheitsindikatoren repräsentiert und wobei jeder erste Regelsatz ausschließlich Regeln zur Auswertung der genannten Krankheitsindikatoren aufweist oder
- bei dem jeder erste Regelsatz eine medizinische Unterdisziplin repräsentiert und wobei jeder erste Regelsatz ausschließlich Regeln aufweist, die wenigstens eine Bedingung an Krankheitsindikatoren von in der genannten medizinischen Unterdisziplin untersuchten Krankheiten aufweist.

3. Computerimplementiertes Verfahren nach Anspruch 1 oder 2, das ferner den Schritt des Anzeigens des ersten Diagnoseergebnisses einem Benutzer aufweist, wobei der genannte Anzeigeschritt Folgendes aufweist:

- Bestimmen (254), ob der Gesamtbewertungswert einen ersten Schwellenwert übersteigt;
- falls der Gesamtbewertungswert den ersten Schwellenwert übersteigt, Ausführen der folgenden Schritte:

  • Anzeigen (255) des ersten Diagnoseergebnisses in einem ersten GUI-Fenster (451, 452, 453);
  • Anzeigen (256) von einem oder mehr Dialogfenstern (454, 455, 456), die einen Benutzer zum Eingeben zusätzlicher Daten des Patienten auffordern;
  • Ermitteln eines endgültigen Bewertungswertes durch Auswerten von einem oder mehr dritten Regelsätzen an den eingegebenen zusätzlichen Daten;
  • Anzeigen (259) eines positiven zweiten Diagnoseergebnisses in einem zweiten GUI-Fenster (457), falls der endgültige Gesamtbewertungswert einen zweiten Schwellenwert übersteigt; und
  • Anzeigen eines negativen zweiten Diagnoseergebnisses, falls der endgültige Bewertungswert den zweiten Schwellenwert nicht übersteigt.

4. Computerimplementiertes Verfahren nach Anspruch 3, bei dem das Verfahren in einem Entscheidungsunterstützungssystem (276) mit einer mehrschichtigen Architektur implementiert wird, bei der eine erste Schicht (274) Zugang zu den biomedizinischen Patientendaten bereitstellt, bei dem eine zweite Schicht (273) eine Regelmaschine aufweist und zum Auswerten der Regeln des ersten Regelsatzes ungeachtet des Krankheitstyps, dessen Vorhandensein zu bestimmen ist, funktionell ist und bei dem eine dritte Schicht (270, 271, 272) die Ergebnisse der ersten und der zweiten Diagnose einem Benutzer anzeigt, bei dem die Anzahl und der Typ von durch die dritte Schicht erstellten Dialogfenstern von einem Identifikator der Krankheit und von den Identifikatoren aller ersten Regelsätze, die einen höchsten Bewertungswert von mehr als 0 haben, abhängt.

5. Computerimplementiertes Verfahren nach einem der Ansprüche 1 bis 4, bei dem das Verfahren zum Herausgeben einer Diagnose eines Patienten als ein Entscheidungsunterstützungssystem (276) auf einem Computersystem (281, 282) implementiert ist, wobei das Entscheidungsunterstützungssystem funktionell mit einer Datenbank (279) gekoppelt ist, die die biomedizinischen Patientendaten des Patienten aufweist, bei dem die Regeln jedes ersten Regelsatzes bei einem aus der Gruppe bestehend aus Folgenden ausgewählten Ereignis an den genannten biomedizinischen Daten ausgewertet werden:

- dem Moment des Startens eines Doktorinformationssystems (287), das funktionell mit dem Entscheidungsunterstützungssystem gekoppelt ist,
- dem Moment des Zugreifens auf die biomedizinischen Patientendaten des Patienten durch das Entscheidungsunterstützungssystem oder durch ein funktionell mit dem Entscheidungsunterstützungssystem gekoppeltes Doktorinformationssystem und
- zu einem vorbestimmten Zeitpunkt und Datum.

6. Computerimplementiertes Verfahren nach einem der Ansprüche 1 bis 6, bei dem das Auswerten der einen oder mehr Regeln jedes ersten Regelsatzes ferner die folgenden Schritte aufweist:

- nach der Auswertung der Regeln Speichern von Informationen, die angeben, dass die Regeln ausgewertet wurden;
- vor der Auswertung der Regeln Prüfen, ob die genannten Informationen bereits gespeichert wurden, und Auswerten der Regeln nur in dem Fall, dass die genannten Informationen noch nicht gespeichert wurden.

7. Computerimplementiertes Verfahren nach einem der Ansprüche 1 bis 6, bei dem die Regeln der ersten Regelsätze von einem Entscheidungsunterstützungssystem aus einem Regelrepositorium (278) empfangen werden, bei dem die Regeln aus dem Regelrepositorium in einem Datenformat gespeichert und zu dem Entscheidungsunterstützungssystem übertragen werden, wobei das genannte Datenformat das Modifizieren oder Austauschen der Regeln ohne Einführen von Änderungen am Entscheidungsunterstützungssystem oder an der Struktur des Regelrepositoriums ermöglicht.

8. Computerimplementiertes Verfahren nach Anspruch 3, bei dem die Regeln des ersten Regelsatzes zusammen mit dem ersten Schwellenwert von einem Entscheidungsunterstützungssystem empfangen werden.

9. Computerlesbares Speichermedium, in dem Anweisungen gespeichert sind, die bei Ausführung durch eine Rechenvorrichtung die Rechenvorrichtung zum Durchführen der Schritte nach Anspruch 1 veranlassen.

10. Rechensystem (278, 279, 281, 282, 283), das Mittel zum Durchführen des Verfahrens nach Anspruch 1 aufweist.

**Revendications**

1. Procédé mis en oeuvre par ordinateur pour déterminer la présence d'une maladie chez un patient, le procédé comprenant :

- la collecte de données biomédicales d'un patient en exécutant les étapes :

• de réception de parties de données biomédicales du patient à partir d'une pluralité de sources de données (283) différentes, ce par quoi, chaque partie reçue de données biomédicales de patient comprend un identifieur unique, l'identifieur unique étant identique dans chacune des parties reçues des données biomédicales de patient,
• de stockage des données biomédicales reçues dans une base de données (279), en utilisant pour ceci l'identifieur en tant que clé d'accès à la base de données pour toutes les parties reçues des données biomédicales de patient, ce par quoi l'identifieur unique est généré par chacune des sources de données en exécutant les étapes de :

- réception (200) d'un secret choisi par l'utilisateur (112) ;
- de stockage du secret choisi par l'utilisateur dans une mémoire (108) ;

- de calcul (202, 204) d'une clé privée (116) en appliquant une intégration (132) et une fonction de randomisation (134) au secret ;
- de stockage de la clé privée dans une mémoire ;
- de calcul (206, 502, 602) d'une clé publique (118) en utilisant la clé privée, ce par quoi le calcul de la clé publique est réalisé en utilisant une fonction cryptographique à sens unique, la clé publique et la clé privée formant une paire de clés cryptographiques asymétriques ;
- de production (208, 316, 504, 604) de la clé publique pour procurer la clé d'accès ; et
- d'effacement du secret et de la clé privée de la mémoire

- la réception (250), par un système informatique (282), de premiers ensembles de règles comprenant des règles, les règles de chaque premier ensemble de règles étant opérationnelles pour évaluer un ensemble d'indicateurs de maladie, les indicateurs de maladie de chaque premier ensemble de règles étant reliés entre eux de manière biomédicale, les règles de chaque premier ensemble de règles étant regroupées en un ou plusieurs deuxièmes ensembles de règles, chaque deuxième ensemble de règles comprenant une valeur de score, ladite valeur de score étant indicatrice du risque du patient d'avoir la maladie dans le cas où l'évaluation de la ou des règles dudit deuxième ensemble de règles concernant les données biomédicales du patient retourne vers un résultat positif, où la totalité des conditions contenues dans certaines règles du ou des deuxièmes ensembles de règles est un sous-ensemble de conditions contenues dans des règles d'un autre ensemble parmi les deuxièmes ensembles de règles d'un premier ensemble de règles particulier ;
- de détermination (251), par le système informatique, pour chaque premier ensemble de règles, de la valeur de score la plus élevée de ses deuxièmes ensembles de règles en évaluant toutes les règles desdits deuxièmes ensembles de règles concernant les données biomédicales du patient ;
- de calcul (252), par le système informatique, d'une valeur de score totale pour la maladie sous la forme d'une dérivée des valeurs de score les plus élevées déterminées de tous les premiers ensembles de règles ;
- la production (253), par le système informatique, d'un premier résultat de diagnostic, le premier résultat de diagnostic étant indicateur de la présence de la maladie chez le patient, le premier résultat de diagnostic étant associé à la valeur de score totale.

2. Procédé mis en oeuvre par ordinateur selon la revendication 1,

- par lequel chaque premier ensemble de règles représente un système d'un organe et dans lequel chaque premier ensemble de règles comprend seulement des règles pour l'évaluation d'indicateurs de maladie devenant manifestes dans ledit système d'organe, ou
- dans lequel chaque premier ensemble de règles représente un ensemble d'indicateurs de maladie reliés de manière biomédicale et dans lequel chaque premier ensemble de règles comprend seulement des règles pour l'évaluation desdits indicateurs de maladie, ou
- dans lequel chaque premier ensemble de règles représente une sous-discipline médicale et dans lequel chaque premier ensemble de règles comprend des règles qui comprennent au moins une condition concernant les indicateurs de maladie de maladies examinées dans ladite sous-discipline médicale.

3. Procédé mis en oeuvre par ordinateur selon la revendication 1 ou 2, comprenant en outre l'étape d'affichage du premier résultat de diagnostic au niveau d'un utilisateur, ladite étape d'affichage comprenant :

- la détermination (254) si la valeur de score totale dépasse une première valeur de seuil ;
- dans le cas ou la valeur de score total n'excède pas la première valeur de seuil, l'exécution des étapes suivantes :

• l'affichage (255) du premier résultat de diagnostic dans une première fenêtre GUI (451, 452, 453) ;
• l'affichage (256) d'une ou de plusieurs fenêtres de dialogue (454, 455, 456) demandant à un utilisateur d'entrer des données additionnelles du patient ;
• le calcul d'une valeur de score final en évaluant un ou plusieurs troisièmes ensembles de règles concernant les données additionnelles entrées ;
• l'affichage (259) d'un deuxième résultat de diagnostic positif dans une deuxième fenêtre GUI (457) dans le cas où la valeur de score finale dépasse une deuxième valeur de seuil, et
• l'affichage d'un deuxième résultat de diagnostic négatif dans le cas où la valeur de score finale ne dépasse pas la deuxième valeur de seuil.

4. Procédé mis en oeuvre par ordinateur selon la revendication 3, où le procédé est mis en oeuvre dans un système d'aide à la décision (276) ayant une architecture à niveaux multiples, par laquelle un premier niveau (274) procure l'accès aux données biomédicales du patient, par laquelle un deuxième niveau (273) comprend un moteur de règles et est opérationnel pour évaluer les règles des premiers ensembles de règles du type de maladie dont la présence doit être déterminée, et par laquelle un troisième niveau (270, 271, 272) affiche les premier et deuxième résultats de diagnostic au niveau d'un utilisateur, par laquelle le nombre et le type de fenêtre de dialogue créées par le troisième niveau dépend d'un identifieur de la maladie et des identifieurs de tous les premiers ensembles de règles ayant une valeur de score la plus élevée supérieure à 0.

5.  Procédé mis en oeuvre par ordinateur selon l'une quelconque des revendications 1 à 4, dans lequel le procédé de production d'un diagnostic d'un patient est mis en oeuvre sous la forme d'un système d'aide à la décision (276) sur un système informatique (281, 282), le système d'aide à la décision étant couplé de manière opérationnelle à une base de données (279) comprenant les données biomédicales de patient du patient, par lequel les règles de chaque premier ensemble de règles sont évaluées par rapport auxdites données biomédicales lorsqu'un évènement étant choisi dans le groupe constitué de :

- le moment de départ d'un système d'information de médecin (287) étant couplé de manière opérationnelle au système d'aide à la décision ;
- le moment de l'accès aux données biomédicales de patient du patient par le système d'aide à la décision ou par un système d'information de médecin étant couplé de manière opérationnelle au système d'aide à la décision ; et
- à une heure et date prédéfinies.

6.  Procédé mis en oeuvre par ordinateur selon l'une quelconque des revendications 1 à 5, dans lequel l'évaluation de la ou des règles de chaque premier ensemble de règles comprend en outre les étapes :

- après avoir évalué les règles, de stockage de l'information indiquant que les règles ont été évaluées ;
- avant l'évaluation des règles, de vérification si ladite information a déjà été stockée et d'évaluation des règles uniquement dans le cas où ladite information n'a pas été encore stockée.

7.  Procédé mis en oeuvre par ordinateur selon l'une quelconque des revendications 1 à 6, dans lequel les règles des premiers ensembles de règles sont reçues par un système d'aide à la décision à partir d'un dépôt de règles (278), ce par quoi les règles sont stockées et transmises à partir du dépôt de règles vers le système d'aide à la décision dans un format de données, ledit format de données permettant de modifier ou d'échanger les règles sans introduire de changements au système d'aide à la décision ou à la structure du dépôt de règles.

8.  Procédé mis en oeuvre selon la revendication 3, dans lequel les règles des premiers ensembles de règles sont produites par un système d'aide à la décision conjointement avec le premier seuil.

9.  Support de stockage lisible par ordinateur ayant des instructions stockées qui, lorsqu'elles sont exécutées par un dispositif informatique, font que le dispositif informatique exécute les étapes selon la revendication 1.

10. Système informatique (278, 279, 281, 282, 283) comprenant des moyens pour la réalisation du procédé selon la revendication 1.

Receiving first rule sets comprising rules, the rules of each first rule set being grouped into one or more second rule sets, each second rule set comprising a score value being indicative of the risk of the patient to have the disease — 250

Determining, for each first rule set, the highest score value of its second rule sets by evaluating all rules of said first rule set on the biomedical data of the patient — 251

Calculating a total score value TSV for the disease as a derivative of the determined highest score values of all first rule sets — 252

Returning a first diagnosis result, the first diagnosis result being indicative of the presence of the disease in the patient, the diagnosis result being assigned with the total score value (TSV) — 253

# Figure 1a

TSV exceeds 1st threshold? — 254

YES

Display a positive first diagnosis result in a first GUI window — 255

Displaying one or more dialog windows prompting a user to enter additional data of the patient — 256

Calculating a final SV, thereby using the entered additional data as input — 257

final SV exceeds 2nd threshold? — 258

No

Display a negative second diagnosis result in a second GUI window — 260

YES

Displaying a positive second diagnosis result in a third GUI window — 259

# Figure 1b

**Figure 2**

**Figure 3a**

**Figure 3b**

254

TSV
exceeds 1st
threshold?

—No——▶  No additional window displayed

YES

451

**◙🖫 Orphan Disease Finder** ▂ X

**MORBUS FABRY**

Because of your specific documentation
for the disease pattern *pain* for this
patient exists a suspicion to a M. Fabry
<u>Literature Reference</u>

Questionnaire                    Remind me later

< 2/2 >

OR

452

**◙🖫 Orphan Disease Finder** ▂ X

**MORBUS FABRY**

Because of your specific documentation
for the disease pattern *nephrology and stroke* for this
patient exists a suspicion to a M. Fabry
<u>Literature Reference</u>

Questionnaire                    Remind me later

< 2/2 >

OR

**◙🖫 Orphan Disease Finder** ▂ X

**MORBUS FABRY**

Because of your specific documentation with
patterns *of multiple organ disease* for this
patient exists a suspicion to a M. Fabry
<u>Literature Reference</u>

453

Questionnaire                    Remind me later

< 2/2 >

# Figure 4a

454

**Questionnaire Pain Characteristics:**

| | true | occasionally true | wrong |
|---|---|---|---|
| Primarily in the extremities? | ● | ○ | ○ |
| Burning pain? | ● | ○ | ○ |
| Absence of inflammation? | ○ | ○ | ○ |
| Since childhood? | ○ | ● | ○ |
| Pain attacs? | ● | ○ | ○ |
| The trigger was sports, heat, feaver? | ○ | ○ | ● |

455

**Questionnaire Pain Characteristics:**

| | true | occasionally true | wrong |
|---|---|---|---|
| Diagnosis of idiopathic renal failure? | ● | ○ | ○ |
| Disease is progressing? | ○ | ○ | ● |
| Parapelvine cysts? | ○ | ○ | ○ |
| Isosthenuria? | ○ | ○ | ● |

456

**Questionnaire Cardiology:**

| | true | occasionally true | wrong |
|---|---|---|---|
| Idiopathic left ventricular hypertrophy? | ○ | ● | ○ |
| Idiopathic cardiac dysrhythmia? | ● | ○ | ○ |
| Concentric left ventricular hypertrophy with hypertrophy of the papillary muscles? | ● | ○ | ○ |

# Figure 4b

⚙️ 💾 **Orphan Disease Finder** ▬ ☒

**MORBUS FABRY**

450 — Based on the current information the presence of Morbus Fabry is unlikely.

**Thank you for your cooperation!**

No ↑

Calculate final SV → Final SV exceeds 2nd threshold? ⌐ 258

257

YES ↓

⚙️ 💾 **Orphan Disease Finder** ▬ ☒

**MORBUS FABRY**

457 — Based on the current information a strong suspect of M. Fabry occurs. For confirmation of the diagnosis, please ....

*Thank you for your cooperation!*

Further information about M. Fabry ...

< 2/2 >

# Figure 4c

Fig. 5

```
┌──────────────────────────────────────────────────────────┐
│   Entering of User selected Secret and public Parameter    │──── 200
└──────────────────────────────────────────────────────────┘
                            │
                            ▼
┌──────────────────────────────────────────────────────────┐
│              Applying embedding Function                   │──── 202
└──────────────────────────────────────────────────────────┘
                            │
                            ▼
┌──────────────────────────────────────────────────────────┐
│             Applying randomizing Function                  │──── 204
└──────────────────────────────────────────────────────────┘
                            │
                            ▼
┌──────────────────────────────────────────────────────────┐
│                  Computing public Key                      │──── 206
└──────────────────────────────────────────────────────────┘
                            │
                            ▼
┌──────────────────────────────────────────────────────────┐
│               Using public Key as Access Key              │──── 208
└──────────────────────────────────────────────────────────┘
```

# Fig. 6

Fig. 7

EP 2 365 458 B1

Entering User selected Secret — 300

Applying a first Hash Function on the Secret
to provide a first Hash-Value — 304

Generating a random Number — 306

Combining the first Hash-Value and the random Number
to provide a Combination — 308

Applying a second Hash Function on the Combination
to provide a second Hash-Value — 310

312

No — Second Hash-Value
fulfills Condition ?

Yes

Computing public Key
using second Hash-Value as private Key — 314

Using public Key as Access Key — 316

# Fig. 8

| Access an Input Value | 500 |
|---|---|

↓

| Calculate an asymmetric cryptographic Key Pair | 502 |
|---|---|

↓

| Output the Public Key of the cryptographic Key Pair as the Access Key | 504 |
|---|---|

# Fig. 9

| Access an Input Value | 600 |

| Calculate an asymmetric cryptographic Key Pair | 602 |

| Output the Public Key of the cryptographic Key Pair as the Access Key | 604 |

| Generate a digital Signature for the Data using the Private Key | 606 |

| Deposit Data and digital Signature into Database using the Access Key | 608 |

| Verify the Authenticity of the Data using the Access Key | 610 |

# Fig. 10

Fig. 11

700  702  710  712  706  708  108  110  112  118  714  716  718  704  736  734  732  726  702  722  728  730  724

Input Secret:

>_

| 1 | 2 ABC | 3 DEF |
| 4 GHI | 5 JKL | 6 MNO |
| 7 PQRS | 8 TUV | 9 WXYZ |
| * | 0 + | # |

EP 2 365 458 B1

52

**Fig. 12**

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070112782 A1
- US 20080263050 A1
- WO 2008085881 A1
- US 20020091687 A1
- US 20090187419 A1
- US 20100131296 A1
- US 20100088320 A1

- US 7742932 B
- US 006754655 B
- US 006212519 B
- EP 09179974 A
- WO 0041613 A2
- US 2006153370 A1

**Non-patent literature cited in the description**

- Biomedical Data: the Acquisition, Storage, and Use. **EDWARD H. SHORTLIFFE ; G. OCTO BARNETT.** Biomedical Informatics: Computer Applications in Healthcare and Biomedicine. Springer, 25 May 2006, 698-736

- **BENJUMEA et al.** *Internet Research,* 2006, vol. 16 (2), 120-139